# EUROPÄISCHE PATENTSCHRIFT

(11) **EP 0 777 689 B1**
(45) Veröffentlichungstag und Bekanntmachung des Hinweises auf die Patenterteilung: **29.03.2000**
(21) Anmeldenummer: 95930443.7
(22) Anmeldetag: 08.08.1995
(51) Int. Cl.: C07K 14/78, C07K 17/02, A61K 38/39, C12N 5/08, A61L 27/00

(54) **VERWENDUNG VON ZELLADHÄSIONS-PEPTIDEN ZUR MODIFIKATION DES HAFTUNGSVERMÖGENS EUKARYONTISCHER ZELLEN UNTEREINANDER**
USE OF CYTADHERENCE PEPTIDES FOR USE IN MODIFYING MUTUAL ADHESION AMONG EUKARYOTIC CELLS
USAGE DES PEPTIDES D'ADHESION CELLULAIRE DESTINES A MODIFIER LE POUVOIR D'ADHESION INTERCELLULAIRE DE CELLULES EUCARYOTES

(30) Priorität: 22.08.1994 DE 4430601
(43) Veröffentlichungstag der Anmeldung: 11.06.1997
(73) Patentinhaber: Beiersdorf Aktiengesellschaft, D-20253 Hamburg (DE)
(72) Erfinder: EICHNER, Wolfram, D-21266 Jesteburg (DE); KOCK, Katharina, D-22869 Schenefeld (DE); MIELKE, Heiko, D-21629 Neu Wulmstorf (DE); DOERSCHNER, Albrecht, D-20357 Hamburg (DE)
(74) Vertreter: UEXKÜLL & STOLBERG
(86) Internationale Anmeldenummer: EP9503135
(87) Internationale Veröffentlichungsnummer: WO9606114

(56) Entgegenhaltungen:
- WO-A-91/18294
- WO-A-92/06199
- WO-A-92/13887
- US-A- 5 026 636
- J. EXP. MED. (1988), 167(2), 718-23 CODEN: JEMEAV;ISSN: 0022-1007, 1988 DALLO, S. F. ET AL 'Identification of P1 gene domain containing epitope(s) mediating Mycoplasma pneumoniae cytadherence'
- Biochemicalien - Organische Verbindingen voor Research en Dignostica Catalogus by Sigma Chemie, 1994 Benelux edition, pages 1076-1078

## Beschreibung

Die vorliegende Erfindung betrifft die Verwendung von Adhäsions-peptiden zur Modifikation des Haftungsvermögens eukaryontischer Zellen untereinander, d.h. deren Verwendung zur Förderung oder Inhibition der Zell/Zell-Adhäsion eukaryontischer Zellen. Die Erfindung betrifft insbesondere die Verwendung der Zelladhäsions-Polypeptide zur Herstellung von Arzneimitteln zur Beeinflussung der Zell/Zell-Interaktionen eukaryontischer Zellen.

Mit dem Begriff (Zell-)Adhäsion/Zelladhäsivität wird im engeren Sinne das Aneinanderheften von Zellen, z.B. bedingt durch die Bindung komplementärer Membranoberflächenmoleküle bezeichnet. Der Begriff "Adhärenz" ist in der Zellkultur gebräuchlich für Zellen, die in vitro an festen Oberflächen binden. Die Begriffe "(Zell-) Adhäsion" und "Adhärenz" werden häufig synonym verwendet.

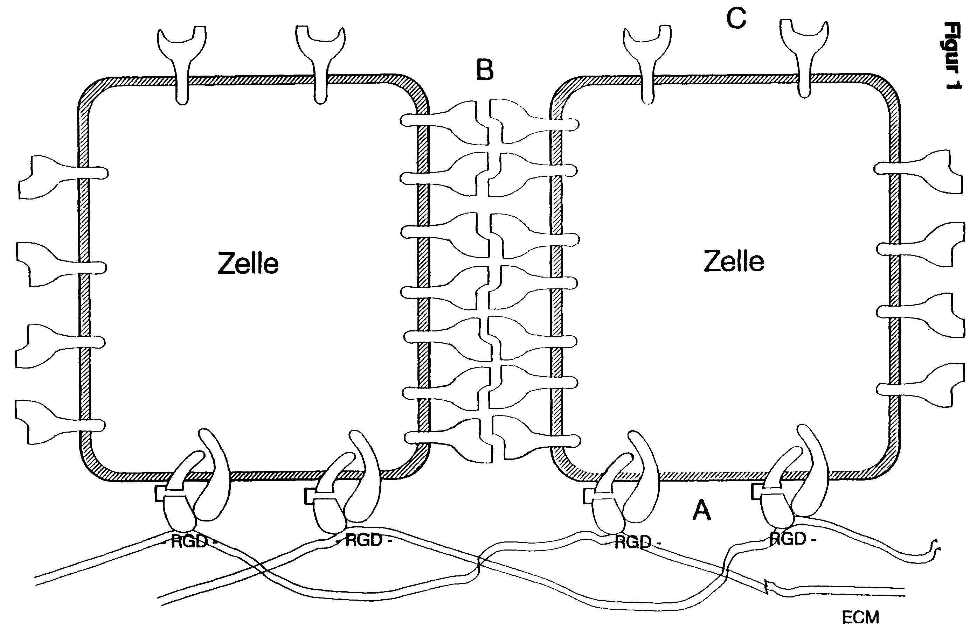

Die Zellen der unterschiedlichen Gewebe oder Organe eines vielzelligen Organismus besitzen eine festgelegte Funktion in einer definierten Umgebung. Bis auf wenige Ausnahmen, wie zum Beispiel die zirkulierenden Zellen der Gefäßsysteme, sind eukaryontische Zellen in der Regel fest verankert. Diese Immobilisierung wird durch zwei Arten der Adhäsion vermittelt, nämlich einerseits durch Zell/Zell-, andererseits durch Zell/Matrix-Interaktionen. Die interzellulären Kontakte, d.h. Zell/Zell-Wechselwirkungen (Fig. 1B), werden insbesondere an der zellulären Basalmembran durch ein als Extrazellularmatrix (ECM) bezeichnetes heterogenes gerüstartiges Netz aus Fasern, das aus Proteinen, Glykoproteinen (zum Beispiel Fibronektine, die Kollagene, Laminin, Thrombospondin und Vitronectin) und anderen Basalmembranassoziierten Makromolekülen (zum Beispiel Proteoglycane und Glukosaminoglucane), d.h. durch Ausbildung von Zell/Matrix-Interaktionen, unterstützt (Fig. 1A). Eine Reorganisation dieser Adhäsions-Strukturen erfolgt beispielsweise im Verlauf von Reparaturprozessen wie der Wundheilung, oder bei der Entstehung und dem Wachstum von Tumoren.

Die zwei Arten der Adhäsion, d.h. die Zell/Zell- und die Zell/Matrix-Interaktionen sind in Figur 1A und 1B schematisch dargestellt. Es ist dieser Abbildung zu entnehmen, daß die Wechselwirkung zwischen Zellen und der Extrazellularmatrix durch verschiedenartige Strukturen vermittelt wird, ähnlich dem "Schlüssel/Schloß-Prinzip". Die Zell/Zell-Adhäsion hingegen findet überwiegend durch Interaktion gleichartiger Zelloberflächenstrukturen, d.h. durch gleichartige Moleküle mit Rezeptorcharakter, statt.

Unter den sogenannten ECM-Faktoren, die die Zell/Matrix-Adhäsion auf der Seite der Basalmembran vermitteln, spielen die Fibronektine eine besondere Rolle. In seiner nativen Form ist das Fibronektinmolekül ein Dimer, das aus identischen Untereinheiten mit einer relativen Molekülmasse Mᵣ von jeweils 220 KD zusammengesetzt ist, die über Disulfidbrücken miteinander verknüpft sind. Ein Merkmal mit besonderer Ausprägung im Fibronektin, wie auch auf anderen proteinogenen ECM-Komponenten, ist die Zusammensetzung aus kurzen repetitiven Aminosäuresequenzeinheiten.

Mehrere dieser zueinander homologen, aber nicht identischen Sequenzen bilden in den einzelnen Fibronektin-Ketten ihrerseits eindeutig abgegrenzte Domänen, die die Bindung an Fibrin, Kollagen oder Heparin vermitteln. Nur eine dieser Domänen besitzt die Fähigkeit, an Zellen zu binden. Der für das Anheftvermögen dieser Fibronektin-Domäne an Zellen verantwortliche Sequenzabschnitt wurde von Pierschbacher et al. (Proc. Natl. Acad. Sci. USA 80 (1983) 1224-1227) identifiziert und sequenziert. Dieser Abschnitt enthält das Tetrapeptid "RGDS" (Arg-Gly-Asp-Ser). Diese Erkennungssequenz wurde von Pierschbacher und Ruoslahti (Nature 309 (1984) 30-33) charakterisiert, indem immer kleiner werdende synthetische Peptide der Zellerkennungsdomäne hinsichtlich ihrer Fähigkeit, das Anheftvermögen von Zellen zu fördern, getestet wurden. Die Sequenz RGD (Arg-Gly-Asp) ist für die Vermittlung der Bindungsaktivität essentiell, während die Aminosäure Serin in Position 4 auch durch andere Aminosäuren ersetzt werden kann (Pierschbacher und Ruoslathi, a.a.O.).

Außer im Fibronektinmolekül wurde auch in zahlreichen anderen Matrix-Proteinen die Sequenz RGDX nachgewiesen, wobei X eine variable, aber nicht beliebige Aminosäure darstellt (vgl. Humphries, J. Cell Sci. 97 (1990) 585-592).

Es ist inzwischen bekannt, daß die Erkennung der RGDX-Sequenz durch Rezeptoren der Zielzellen gewährleistet ist und es sich hierbei um hochspezifische Interaktionen zwischen Rezeptoren auf der Zelloberfläche und der Matrix handelt. Die als Integrine bezeichneten Rezeptor-Moleküle sind Heterodimere, die aus α- und β-Untereinheiten (Fig. 1A) aufgebaut sind. Auf Seiten des mit den Integrinen in Wechselwirkung tretenden Liganden wird die Spezifität der adhärenzwirksamen RGDX-Sequenz durch die unmittelbar benachbarten Sequenzabschnitte bewirkt (Pierschbacher und Ruoslathi, J. Biol. Chem. 262 (1987), 17294-17298). Dies hat zur Folge, daß ECM-Komponenten trotz gleicher RGD-Kernsequenz mit Integrin-Heterodimeren ganz unterschiedlicher Komposition, d.h. mit speziellen Rezeptoren, interagieren (vgl. für eine Übersicht zum Beispiel Dedhar, Bio Essays, 12 (1990), 583-590 und Hynes, Cell 69 (1992), 11-25).

Die hochkonservierte Aminosäuresequenz des Tripeptids "RGD" wurde außer in Proteinen der Extrazellularmatrix auch in verschiedenen bakteriellen und viralen Proteinen nachgewiesen, und ihre adhärenzvermittelnde Funktion in diesen Proteinen wurde experimentell belegt. Sowohl Pertactin, ein Oberflächenprotein aus *Bordetella pertussis* (Leininger et al., Proc. Natl. Acad. Sci. USA 88 (1991) 345-349), als auch das "tat" (Type 1 Transactivation Protein) des humanen *HIV-1* Virus (Brake et al., J. Cell Biol. 111 (1990) 1275-1281) sowie ein Adenovirus-Oberflächenprotein (Nemerow et al., Trends in Cell Biol. 4 (1994) 52-55) enthalten das RGD-Sequenzmotiv.

Den im Stand der Technik bekannten Adhäsionsproteinen und darauf identifizierten Peptidsequenzen ist gemeinsam, daß sie die Interaktionen zwischen Zelle und Matrix vermitteln bzw. die Zell/Matrix-Adhäsion fördern. Dies gilt auch für die WO92/13887. Neben den Interaktion mit der Extrazellularmatrix treten Zellen untereinander ebenfalls in Wechselwirkung, wobei diese Zell/Zell-Adhäsion beiderseits durch spezifische Oberflächenrezeptoren bewirkt wird. Das heißt, daß im Unterschied zur Zell/Matrix-Interaktion, bei der ECM-Komponente(n) und Zelloberflächen-Rezeptor(en) nach einem "Schlüssel-Schloß-Prinzip" miteinander in Wechselwirkung treten, bei der Zell/Zell-Adhäsion in der Regel gleichartige Moleküle (d.h. Moleküle mit Rezeptorcharakter) an der Interaktion beteiligt sind.

Die Untersuchung von sogenannten Adhäsionsfaktoren ist von großem Interesse, da ihre zentrale Rolle bei allen Vorgängen der Zell/Zell-Interaktion oder der Zellwanderung immer deutlicher wird (Travis, Science 260, (1993), 906-908). In der embryonalen Entwicklung werden Adhäsionsmoleküle für die Ausbildung von Geweben und Organen benötigt. Daneben sind diese Faktoren beispielsweise maßgeblich an Prozessen der Wundheilung, entzündlichen Prozessen und der Metastasierung von Krebszellen beteiligt.

Aufgabe der vorliegenden Erfindung ist es daher, die interzellulären Wechselwirkungen eukaryontischer Zellen durch Adhäsions-Peptide effektiv zu beeinflussen, d.h. durch Adhäsions-Peptide, die das Haftungsvermögen zwischen eukaryontischen Zellen, wie z.B. von Endothelialzellen oder humanen epidermalen Zellen, untereinander modifizieren. Derartige Peptide sollen auf einfache Weise erhältlich sein und sowohl zur Förderung als auch zur Inhibition der Zell/Zell-Adhäsion eukaryontischer Zellen verwendet werden können. Insbesondere sollen die Peptide außer zur in vitro-Modifikation des Haftungsvermögens zwischen eukaryontischen Zellen untereinander, wie zum Beispiel zur Herstellung von Hauttransplantaten sowie künstlichen Blutgefäßen, auch zur Herstellung von Arzneimitteln zur Beeinflussung der Zell/Zell-Adhäsion eukaryontischer Zellen, beispielsweise zur Förderung der Anwachsrate von Hauttransplantaten oder Organtransplantaten, verwendet sowie für die oben ferner genannten Anwendungen eingesetzt werden können.

Die Aufgabe wird erfindungsgemäß durch den Gegenstand der Ansprüche 1 bis 39 gelöst.

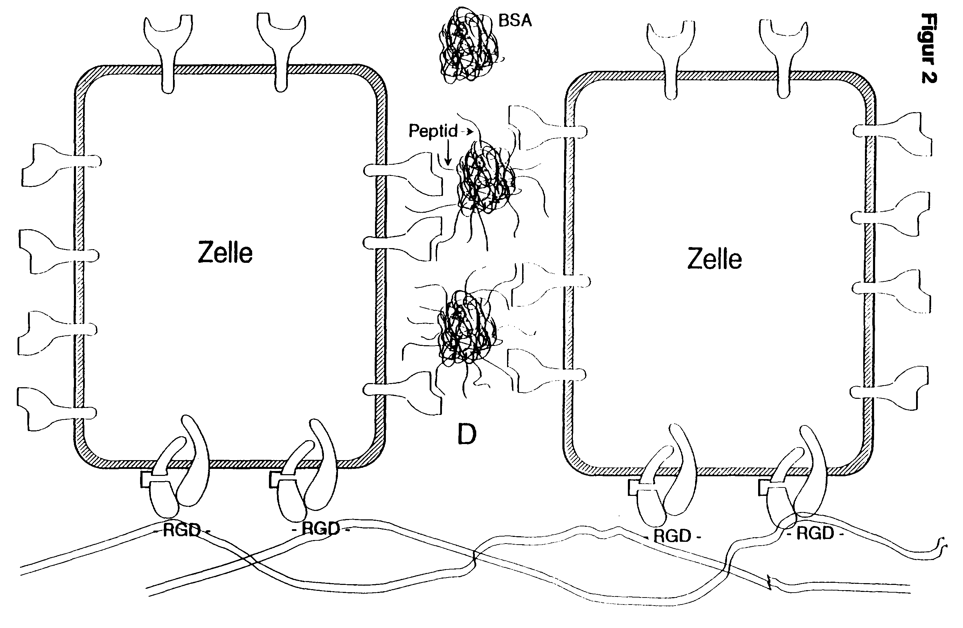

Die im Rahmen der vorliegenden Erfindung verwendeten Peptide sind in der Lage, interzelluläre Adhäsionsvorgänge, d.h. Zell/Zell-Interaktionen, wirksam zu beeinflussen, und sind somit äußerst nützliche und vielseitig einsetzbare Hilfsmittel. In einer immobilisierten Form, zum Beispiel durch Kopplung an Trägermoleküle, können sie als Substrat für eine Vielzahl von Zelltypen eingesetzt werden, beispielsweise um allgemein Zellanheftungs- oder Wundheilungsprozesse zu fördern. Sie können als Bestandteil resorbierbarer Wundauflagen die Kolonialisierung von Wunden, wie zum Beispiel Verbrennungswunden, über die Förderung der Zellmigration auf effektive Weise beschleunigen sowie ferner als Adhärenz-Substrat für gefäßauskleidende Endothelialzellen bei der Entwicklung von Gefäßersatz eingesetzt werden. Der Wirkmechanismus Zell-Zell-Kontakte vermittelnder Substratgekoppelter Adhäsionspeptide ist schematisch in Fig. 2D dargestellt.

Galenische Zubereitungen adhäsionswirksamer Peptide können in besonders bevorzugter Weise auch zur Förderung der Anwachsrate von Hauttransplantaten (skin grafts) dienen. Bislang kommt es bei etwa 40 % der Transplantationen, im Fall von Verbrennungen sogar bis zu 60 %, zu einer sogenannten "graft-failure", bei der das Transplantat nicht anwächst. Durch Einsatz interzellulär adhäsionswirksamer Peptide können die Interaktionen zwischen Transplantat und Wundbett wirksam gefördert werden, wodurch die Wahrscheinlichkeit für Transplantationserfolge erheblich gesteigert werden kann. Ferner ist auch die Verwendung bei der Herstellung von Hauttransplantaten möglich. Ferner können Konjugate aus adhäsionswirksamen Peptiden und pharmazeutischen Wirkstoffen hergestellt werden, wodurch erreicht werden kann, daß die Wirkstoffe infolge der hochspezifischen Bindungsaffinität des Peptidanteiles gezielter an Rezeptoren der Zielzelle herangebracht werden.

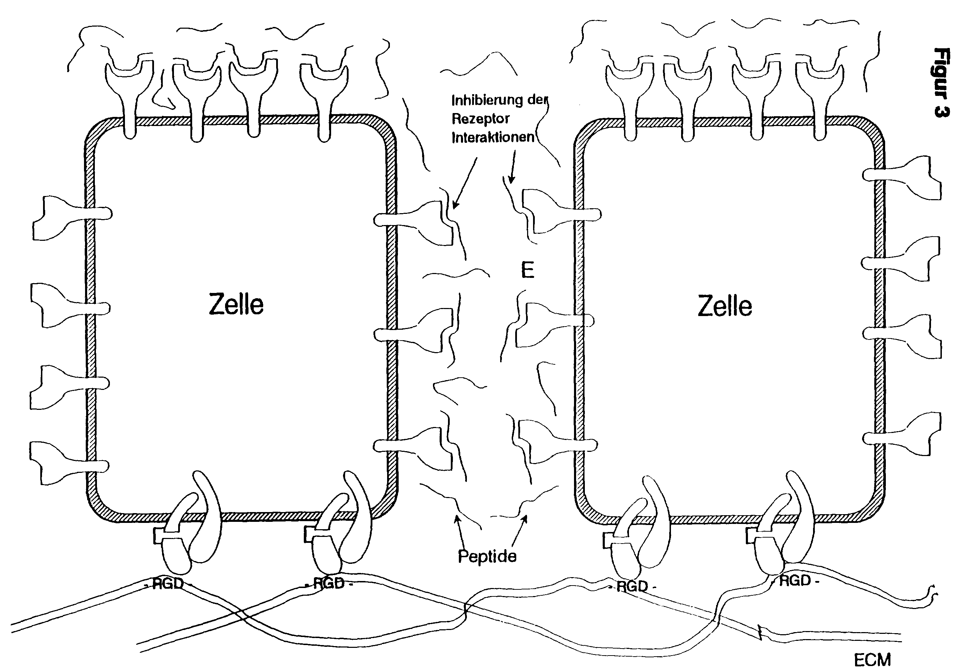

In einer löslichen Form sind mit derartigen Faktoren auch gegenteilige Effekte, also die Inhibition der Zellanheftung, erreichbar, da ein kompetitiver Hemmeffekt durch spezifische Absättigung von Oberflächenrezeptoren eintritt (in Fig. 3E schematisch dargestellt). Bei einer Reihe von pathologischen Prozessen, wie zum Beispiel arteriosklerotischen oder thrombotischen Läsionen, kommt es als Folge übermäßig stimulierter Adhäsionsvorgänge an den Gefäßoberflächen zur Gefäßverengung bis hin zum Gefäßverschluß. Die erfindungsgemäß verwendeten Adhäsions-inhibierenden Peptide können einem derartigen pathologischen Prozeß in spezifischer Weise entgegenwirken. Eine Inhibition der Zellanheftung ist besonders erwünscht, um entzündliche Vorgänge abzuschwächen, wobei der Rezeptor-vermittelte Eintritt von Entzündungszellen aus dem Gefäßsystem in das Gewebe gezielt beeinflußt werden kann. Andere Einsatzmöglichkeiten für Adhäsions-inhibitorisch wirksame Peptide sind die Prophylaxe von Arteriosklerose und Thrombose. Adhäsions-Peptide können ferner in kosmetisch-/dermatologischen Zubereitungen, beispielsweise als Mittel zur Verhinderung großflächiger Kopf- oder Hautschuppen, eingesetzt werden.

Gegenstand der vorliegenden Erfindung ist somit die Verwendung eines Peptids, das die Aminosäuresequenz

AS3-AS2-AS1

aufweist, wobei
AS1 Gly, Pro oder Asp ist,
AS2 Asp, Leu, Asn oder Ser ist
AS3 Leu, Ile, Phe oder Gly ist, und
und wobei
AS1 die endständige Aminosäure am carboxyterminalen Ende des Peptids bezeichnet, wobei die Aminosäuresequenz am carboxyterminalen Ende der Peptide um bis zu zwei beliebige Aminosäuren verlängert sein kann und wobei die Aminosäuresequenz aminoterminal an AS3 bis zu einer Kettenlänge von 30 Aminosäuren verlängert sein kann,
ausgenommen Peptide, die am carboxyterminalen Ende die Sequenz Phe-Ser-Pro oder Phe-Asp-Gly enthalten,
zur Herstellung einer pharmazeutischen Zusammensetzung zur Modifikation des Haftungsvermögens eukaryontischer Zellen untereinander.

Unter Modifizieren des Haftungsvermögens eukaryontischer Zellen untereinander wird erfindungsgemäß verstanden, daß die interzelluläre Adhäsion eukaryontischer Zellen entweder gesteigert oder inhibiert wird.

Die Aminosäuren sind entsprechend ihrem Drei-Letter-Code (bzw. Ein-Letter-Code) bezeichnet. Im einzelnen bedeuten: Ala (bzw. A) Alanin, Arg (bzw. R) Arginin, Asn (bzw. N) Asparagin, Asp (bzw. D) Asparaginsäure, Cys (bzw. C) Cystein, Gln (bzw. Q) Glutamin, Glu (bzw. E) Glutaminsäure, Gly (bzw. G) Glycin, His (bzw. H) Histidin, Ile (bzw. I) Isoleucin, Leu (bzw. L) Leucin, Lys (bzw. K) Lysin, Met (bzw. M) Methionin, Phe (bzw. F) Phenylalanin, Pro (bzw. P) Prolin, Ser (bzw. S) Serin, Thr (bzw. T) Threonin, Trp (bzw. W) Tryptophan, Tyr (bzw. Y) Tyrosin und (bzw. V) Val Valin.

Im Rahmen der vorliegenden Erfindung können die oben genannten Aminosäuren auch durch modifizierte Aminosäuren, wie z.B. Hydroxylysin (Hyl) anstelle von Lysin, ersetzt werden. Entsprechend modifizierte oder seltene Aminosäuren, die erfindungsgemäß verwendet werden können, sind in WIPO-Standard ST. 23 (Amtsblatt des Europäischen Patentamts 12/1992, S. 9 ff., Punkt 12) aufgeführt. Bei Aminosäuren, die als optische Isomere vorkommen, können sowohl die D- als auch die L-Formen verwendet werden, wobei erfindungsgemäß die L-Isomere bevorzugt sind.

Unter dem Begriff "Adhäsions-Peptid" bzw. "Peptid" werden im Rahmen der vorliegenden Erfindung allgemein Aminosäuresequenzen mit mindestens drei Aminosäuren verstanden, die über die üblichen Peptid-Bindungen (Amid-Bindungen) miteinander verknüpft sind. Die Kettenlänge dieser Aminosäureketten kann variieren, wobei aminoterminal (d.h. an AS3) verlängerte Peptide (bzw. Oligo- oder Polypeptide) mit bis zu 30, vorzugsweise 20, Aminosäuren bevorzugt sind. Die erfindungsgemäß verwendeten Adhäsions-Peptide liegen vorzugsweise in linearer Form vor, es sind jedoch auch verzweigte Aminosäureketten denkbar, ohne daß der die Zell/Zell-Adhäsion beeinflussende Effekt dadurch zwangsläufig vermindert wird.

Gemäß einer bevorzugten Ausführungsform der Erfindung werden Peptide verwendet, die mindestens die Aminosäuresequenz

AS4-AS3-AS2-AS1

(SEQ ID NO: 34) aufweisen, wobei
AS1, AS2 und AS3 die oben angegebene Bedeutung haben und AS4 Leu oder Ser ist.

Besonders bevorzugt werden Peptide verwendet, die mindestens die Aminosäuresequenz

AS5-AS4-AS3-AS2-AS1

(SEQ ID NO: 35) aufweisen, wobei
AS1, AS2, AS3 und AS4 die oben angegebene Bedeutung haben und AS5 Glu, Ser, Asp oder Asn ist.

Die erfindungsgemäß verwendeten Peptide enthalten gegebenenfalls vorzugsweise Glutaminsäure (Glu) und/oder Glycin (Gly) am carboxyterminalen Ende. Gemäß einer besonderen Ausführungsform der vorliegenden Erfindung bildet AS1 die endständige Aminosäure am carboxyterminalen Ende des Peptids.

Die erfindungsgemäß verwendeten Adhäsions-Peptide sind sowohl durch charakteristische Sequenzmerkmale im Bereich des Carboxyterminus als auch im aminoterminalen Bereich gekennzeichnet. Es hat sich gezeigt, daß unter den oben aufgeführten, mit den allgemeinen Formeln definierten Peptiden insbesondere die Verwendung von Adhäsions-Peptiden mit 6 bis 30 Aminosäuren, vorzugsweise 12 bis 14 Aminosäuren, von Vorteil ist, deren Aminosäure am aminoterminalen Ende des Peptids Gly, Ser, Leu, Ile, Arg oder Thr ist. Das Haftungsvermögen zwischen eukaryontischen Zellen läßt sich besonders effektiv modifizieren, wenn die aminoterminale Aminosäure Gly ist.

Den Aminosäuren in den Positionen 3 und 5 vom carboxyterminalen Ende (AS3 bzw. AS5) kommt ebenfalls besondere Bedeutung bei der Beeinflussung der Zell/Zell Adhäsion eukaryontischer Zellen zu, und in vorteilhafter Weise werden die Aminosäuren Leu (als AS3) und/oder Glu (als AS5) eingesetzt. Bei Peptiden mit einer Länge von mindestens 5 Aminosäuren wird die gleichzeitige Verwendung von Leu und Glu in den Positionen 3 und 5 am meisten bevorzugt.

Es konnte ferner gezeigt werden, daß bereits das Tripeptid Leu-Asp-Gly (AP#27) das Haftungsvermögen eukaryontischer Zellen untereinander wirksam beeinflußt. Diese Aminosäurefolge findet sich auch in dem Pentapeptid Glu-Leu-Leu-Asp-Gly (AP#25) sowie im Heptapeptid Leu-Ala-Glu-Leu-Leu-Asp-Gly (AP#21) wieder, die die interzelluläre Adhäsion eukaryontischer Zellen ebenso wirksam beeinflussen können wie das oben genannte Tripeptid Leu-Asp-Gly. Das Pentapeptid, das in den Positionen 4 und 5 (AS4 und AS5) die Aminosäuren Leu und Glu aufweist (AP#25), ist im Hinblick auf die Modifikation des Haftungsvermögens eukaryontischer Zellen untereinander gegenüber dem Tripeptid sogar noch etwas wirkungsvoller, was den vorteilhaften Einfluß der letztgenannten Aminosäuren in Peptiden mit einer Länge von mindestens fünf Aminosäuren belegt.

Die Peptide und die die carboxyterminal um ein bzw. zwei Aminosäuren verlängerte Sequenz das Heptapeptids AP#21 umfassen, zeigen eine im Vergleich zu AP#21 erhöhte adhärenzfördernde Wirkung.

Als im Rahmen der vorliegenden Erfindung verwendete Adhäsions-Peptide mit 12 bis 14 Aminosäuren hat sich die Sequenz als besonders wirksam erwiesen. Die Deletion der Aminosäure Leu in Position 7 vom carboxyterminalen Ende des letztgenannten Oligopeptids führt zu dem besonders wirkungsvollen Dodecapeptid das einen besonders effektiven, die interzelluläre Adhäsion eukaryontischer Zellen beeinflussenden Effekt aufweist. Beide Adhäsions-Peptide sind sowohl durch die aminoterminale Aminosäure Gly als auch durch die Aminosäuresequenz Glu-Leu-Leu-Asp-Gly (vgl. SEQ ID NO: 25) gekennzeichnet.

Die Untersuchung der Peptide AP#7, AP#14, AP#16 und AP#24, die in Aminosäureposition 7 (AS7) kein Leucin (Leu) aufweisen, zeigen, daß diese Aminosäure an der betreffenden Sequenzposition keinen positiven, d.h. adhärenzfördernden Einfluß hat. Erfindungsgemäß bevorzugt ist daher die Verwendung der aminoterminalen Sequenz

Weitere, das Haftungsvermögen eukaryontischer Zellen untereinander sehr effektiv modifizierende Adhäsions-Peptide mit 12 bzw. 18 Aminosäuren sind sowie

Die im Rahmen der vorliegenden Erfindung verwendeten Adhäsions-Peptide lassen sich auf einfache Weise synthetisch herstellen, vorzugsweise automatisiert, d.h. unter Verwendung eines im Handel erhältlichen Peptid-Synthesizers. Für kleinere Fragmente ist ferner der Rückgriff auf natürliche Oligo- oder Polypeptide denkbar, wobei jedoch der automatisierten Herstellung der Adhäsions-Peptide aufgrund der Einfachheit, der Präzision und des Reinheitsgrades dieser Synthese der Vorzug zu geben ist.

Die erfindungsgemäß verwendeten Adhäsions-Peptide eignen sich sowohl zur Förderung als auch zur Inhibition bzw. Verminderung (Reduktion) der Zell/Zell-Adhäsion eukaryontischer Zellen. Werden beispielsweise Zellen mit den oben genannten Peptiden in Kontakt gebracht, kommt es aufgrund einer Konkurrenzreaktion mit den natürlichen rezeptorähnlichen Oberflächenstrukturen der eukaryontischen Zellen zur Reduktion des Haftungsvermögens, d.h. zu einer Hemmung bzw. Inhibition der Zell/Zell-Adhäsion.

Gemäß einer besonderen Ausführungsform der Erfindung können die Adhäsions-Peptide auch an Träger gebunden verwendet werden. Voraussetzung für die Auswahl eines geeigneten Trägermoleküls ist, daß weder durch den Träger, noch durch den Kopplungsvorgang die funktionelle biologische Aktivität des/der Peptide beeinträchtigt wird. Träger- oder Carriermoleküle im erfindungsgemäßen Sinne können ganz unterschiedlichen Stoffklassen angehören. Aus der Stoffklasse der Kohlenhydrate/Polysaccharide wären beispielsweise Glycane wie Stärke, Glykogen, Cellulose, Pektin, Amylose, Dextran, Polysucrose oder Chitosan einsetzbar. Ebenso können Glukosaminoglycane wie Heparin, Chitin, Chondroitinsulfat, Hyaluronsäure oder Lipopolysaccharide verwendet werden.
Einige ausgewählte Beispiele für die Stoffklasse der Proteine/Polypeptide sind Komponenten der ECM wie z.B. Kollagen, Immunoglobuline, BSA, KLH (Keyhole Limpet Hemocyanin), Ovalbumin, Polylysin oder Lipoproteine. Weiterhin ist es möglich, die patentgemäß verwendeten Peptide an synthetische Polymere, z.B. Polyethylenglykol (PEG) oder Polyvinylalkohol, an (Phospho-) Lipide wie beispielsweise Ceramide oder Paraffin sowie an Alkohole, z.B. Glycerol zu konjugieren.
Die chemische Kopplung erfolgt in der Regel direkt über reaktive Gruppen des Trägers und des Peptides, oder mit Hilfe sog. "Linker" (Verbindungsmoleküle). Erfindungsgemäß wurde für die Kopplung der Peptide an BSA der bifunktionelle Linker Sulfosuccinimidyl-4-(N-maleimidomethyl)-cyclohexan-1-carboxylat (Sulfo-SMCC) eingesetzt. Die kopplungsfähigen Gruppen können auf Träger und Peptid vorhanden sein, aber auch durch Aktivierung einer reaktiven Gruppe in das Molekül eingeführt werden. Gebräuchliche reaktive Funktionen sind beispielsweise die Amino- (NH₂), Imino- (Imidazolring), Hydroxyl- (OH), Sulfhydryl- (SH), oder Carboxyl- (COOH) Gruppen.

Ein vorteilhaftes und patentgemäß eingesetztes Verfahren ist die Kopplung der mit einem carboxyterminalen Cystein versehenen Peptide an das Trägermolekül (BSA), welches zuvor mit dem Linker Sulfo-SMCC aktiviert wurde.

Soll das Peptid-/Träger-Konjugat adhäsionsfördernde Eigenschaften besitzen, so müssen wenigstens zwei Peptid-Moleküle an einen einzelnen Carrier gebunden sein. Trägermoleküle, an welche kopplungsbedingt nur ein einzelnes Peptid gebunden ist, besitzen ähnliche Bindungseigenschaften wie freie, d.h. nicht an einen Carrier gebundene Peptide. Sie wirken, ähnlich wie für freie Peptide in Figur 3E beschrieben, über die Absättigung von Epitopen auf den Rezeptormolekülen adhäsions-inhibitorisch. Die so modifizierten Zellen sind schließlich nicht mehr oder nur noch in stark vermindertem Maße in der Lage, mit benachbarten eukaryontischen Zellen zu adhärieren.

Wenn mindestens zwei Adhäsions-Peptide an eine einzelne Trägereinheit gebunden sind, findet hingegen eine Förderung des Adhäsionsvermögens eukaryontischer Zellen untereinander statt, da jeweils ein Träger-gekoppeltes Peptid an eine Zelle adhäriert und ein anderes Peptid an demselben Träger die Adhäsion zu einer Nachbarzelle vermittelt. In praxi werden je nach Art des verwendeten Trägers vorzugsweise wesentlich mehr als zwei Peptide an eine einzelne Trägereinheit gebunden sein (etwa 20 bei BSA), um die Zell/Zell-Adhäsion effektiv zu fördern.

Die im Rahmen der vorliegenden Erfindung verwendeten Adhäsions-Peptide sind geeignet, die interzellulären Wechselwirkungen beliebiger eukaryontischer Zellen untereinander zu fördern oder zu hemmen, d.h. es gibt praktisch keine Beschränkung hinsichtlich der eukaryontischen Zielzellen. So kann das Haftungsvermögen von dermalen und epidermalen Zellen zu entsprechend benachbarten Zellen beispielsweise ebenso wirksam modifiziert werden wie dasjenige von Endothelialzellen, differenzierten Zellen bestimmter Organe (wie zum Beispiel Leberzellen, Nierenzellen usw.) oder von Krebszellen jeweils untereinander oder zu anderen eukaryontischen Zellen.

Die erfindungsgemäß verwendeten Adhäsions-Peptide können folglich für die unterschiedlichsten in vitro- und in vivo-Anwendungen eingesetzt werden, bei denen die Adhäsion bzw. das Haftungsvermögen eukaryontischer Zellen untereinander eine Rolle spielt. In diesem Zusammenhang wird auch auf die oben im Zusammenhang mit der Aufgabe der vorliegenden Erfindung genannten Anwendungsgebiete verwiesen, für die sich die erfindungsgemäß verwendeten Adhäsions-Peptide in vorteilhafter Weise eignen.

Insbesondere sind die im Rahmen der vorliegenden Erfindung verwendeten Peptide beispielsweise zur Herstellung künstlicher Blutgefäße geeignet, wobei die Adhäsion der gefäßauskleidenden Endothelialzellen untereinander gefördert wird. Die bei Verwendung der Adhäsions-Peptide bewirkte Steigerung des Haftungsvermögens von epidermalen Zellen kann vor allem zur Herstellung von Hauttransplantaten genutzt werden, wobei man entsprechende, bereits im Stand der Technik für diesen Zweck verwendete Kulturgefäße mit den Peptiden beschichtet, um die Bildung eines gleichmäßigen künstlichen Hauttransplantats zu fördern. Für die Anwendungen, bei denen die Peptide das Haftungsvermögen positiv beeinflussen sollen, d.h. in den Fällen, in denen die Zell/Zell-Interaktion gefördert werden soll, liegen die Adhäsions-Peptide an Träger gebunden vor, wobei mindestens zwei Peptide an jeweils eine Trägereinheit gebunden sind. In besonders bevorzugter Weise und je nach Art des verwendeten Trägers können sehr viel mehr Peptide an ein und denselben Träger gebunden sein.

In Fällen, in denen eine Adhäsion zwischen eukaryontischen Zellen unerwünscht ist, d.h. in Fällen, in denen man vorzugsweise isolierte Zellen oder Einzelzellsuspensionen benötigt, können die Adhäsions-Peptide ebenfalls erfolgreich verwendet werden. Dabei werden zur Absättigung der adhäsionswirksamen Oberflächenstrukturen der Zellen entweder die isolierten Peptide eingesetzt oder Peptid-Träger-Konjugate verwendet, bei denen jeweils nur ein Peptid an eine Trägereinheit gebunden ist.

Wundheilungsvorgänge der Haut verlaufen in zwei voneinander abgrenzbaren Prozessen: Kontraktion und Epithelialisierung. Letzterer Prozess betrifft nur die epithelialen Zellagen. Etwa 24 Stunden nach der Verwundung beginnt die Migration der Keratinozyten, die sich bis zum Wundverschluß fortsetzt (Peacock, 1984, Wound Repair 3rd Ed., W.B. Saunders Co, Philadelphia). Substanzen, die als chemotaktische Attraktoren oder Lockstoffe für epidermale Zellen wirken, können die Epithelialisierung von Wunden und damit Wundheilungsvorgänge in der Haut beschleunigen. Erfindungsgemäß kommt eine Verwendung der Peptide als chemotaktischer Attraktor in Betracht, da die chemotaktische Wirkung beispielhaft für einige Peptide nachgewiesen werden konnte.

Die in vivo-Verwendung der Adhäsions-Peptide ist ebenso vielseitig wie der Einsatz in vitro, da mit den Peptiden praktisch überall dort im menschlichen oder tierischen Organismus eingegriffen werden kann, wo Adhäsionsvorgänge zwischen eukaryontischen Zellen eine Rolle spielen. So läßt sich mit den Adhäsions-Peptiden beispielsweise die Anwachsrate von Haut- oder Organtransplantaten (wie z.B. Leber oder Niere) steigern, aber auch die Wundheilung läßt sich durch die Beeinflussung der Zell/Zell-Interaktionen wirksam fördern. Die Inhibition der Zell/Zell-Adhäsion hingegen spielt zum Beispiel bei der Thrombose- und/oder der Thrombose- und/oder Arterioskleroseprophylaxe sowie bei der Unterdrückung der Metastasierung von Krebszellen eine wichtige Rolle, also in Fällen, in denen die Adhäsion eukaryontischer Zellen untereinander unerwünscht ist, da sie zu krankhaften oder in der Folge sogar tödlichen Veränderungen im Organismus führen. Mit Hilfe der im Rahmen der vorliegenden Erfindung verwendeten Adhäsions-Peptide läßt sich die Bildung derartiger Zell-Anhäufungen wirksam reduzieren bzw. vollständig unterdrücken.

Für die in vivo-Anwendung liegen die Adhäsions-Peptide vorzugsweise in Form galenischer Zubereitungen vor, d.h in Form von Arzneimitteln, die neben den erfindungsgemäß verwendeten Peptiden übliche Trägerstoffe, Hilfsmittel und/oder Zusatzstoffe enthalten, wobei die Peptide entweder nicht an die oben genannten Träger geknüpft sind oder - je nachdem, ob das Arzneimittel das Haftungsvermögen eukaryontischer Zellen untereinander hemmen oder fördern soll - ein oder mindestens zwei Peptide an eine Trägereinheit gebunden sind. Für den Fall, daß die pharmazeutische Zusammensetzung zur Förderung der Zell/Zell-Adhäsion vorgesehen ist, können die an jeweils eine Trägereinheit gebundenen Adhäsions-Peptide untereinander jeweils gleich oder verschieden sein.

Ferner eignen sich die erfindungsgemäß verwendeten Peptide auch zur qualitativen und/oder quantitativen Bestimmung der Zell/Zell-Adhäsion eukaryontischer Zellen (in vitro und in vivo).

Die vorliegende Erfindung wird nachfolgend anhand von Beispielen beschrieben.

### Beispiele

Alle hier untersuchten Peptidsequenzen sowie davon abgeleitete, modifizierte Varianten dieser Sequenzmuster und alle Kontrollpeptide wurden durch Peptidsynthese hergestellt. Als Positivkontrollen wurden synthetische, adhäsionswirksame Peptide aus ECM-Molekülen eingesetzt. Beide als Vergleich getestete Fibronektin (FN)-Fragmente IVA1 und IVA1b (Bezeichnung analog W084/00540) enthalten das Sequenzmuster Arginin-Glycin-Asparaginsäure (Arg-Gly-Asp oder RGD), (Pierschbacher und Ruoslahti, Nature 309 (1984) 30-33) und ermöglichen den Nachweis, daß die erfindungsgemäß verwendeten Peptide im Gegensatz zu den die Zell/Matrix-Interaktion beeinflussenden sogenannten RGD-Peptiden die Interaktionen eukaryontischer Zellen wirksam beeinflussen. Ebenfalls getestet wurde die Aminosäuresequenz SVTLG. Die Sequenz SVTXG (X = variable Aminosäure) ist im humanen Thrombospondin, das bei der Thrombocytenaggregation eine bedeutende Rolle spielt (vgl. Prater et al., J. Cell Biol. (1991) 1031-1040). Als weitere Positivkontrolle diente ein als P-15 bezeichnetes adhäsionsfördendes Fragment der α-1(I)-Kette des Kollagens (WO 91/02537).

Es ist im Stand der Technik bekannt, daß unterschiedliche physikalische Eigenschaften von Peptiden Auswirkungen auf das Aufzugsvermögen dieser Peptide auf die Oberfläche eines Kulturgefäßes haben können. Allen erfindungsgemäß getesteten Peptiden wurde daher die zusätzliche Aminosäure "Cystein" am Carboxyterminus angehängt. Die SH-Funktion des Cysteins wird dazu benötigt, die Peptide an entsprechende Carriermoleküle zu koppeln. Durch entsprechende Konjugation der Peptide an Carriersubstanzen werden biophysikalische Unterschiede weitgehend kompensiert.

### 1. Peptidsynthese und Charakterisierung

Alle getesteten Peptide wurden nach der Festphasen-Synthesemethode nach Merrifield (R.B. Merrifield J. Am. Chem. Soc. 85 (1963) 2149-2154; vgl. auch Fields & Noble, Int. J. Protein Res. 35 (1990) 161-214) unter Verwendung von 9-Fluorenylmethoxycarbonyl (Fmoc) Aminosäuren auf einem 9050 Peptidsynthesegerät der Fa. MilliGen synthetisiert. Von Aminosäuren mit verschiedenen optischen Isomeren wurde in den folgenden Beispielen ausschließlich die L-Formen beschrieben, wobei die Verwendung der D-Isomere die Adhäsionswirksamkeit der Peptide nicht oder nur unwesentlich beeinflußt. Die Abspaltung der Schutzgruppen und Freisetzung der Peptide erfolgte nach den Herstellerangaben entweder mit Trifluoressigsäure (TFA)/Thioanisol/Ethandithiol/Anisol (90 %/5 %/3 %/2 %) oder mit TFA/Phenol (95 %/5 %). Die Reinigung erfolgte durch einen linearen Gradienten von Puffer A nach Puffer B (Puffer A: 0,1 % TFA, Puffer B: 90 % Acetonitril in Puffer A) an einer VYDAC C₁₈ HPLC-Säule (Dimension 250 x 30mm) bei einer Flußrate von 9 ml/min. Anschließend wurden die Peptide in einer Hetosicc Gefriertrocknungsanlage der Fa. NUNC bei < 0,04 mbar lyophilisiert.

Die Aufklärung der Aminosäuresequenzen erfolgte durch Proteinsequenzanalyse auf einem Gasphasensequenzierautomaten Modell 477A der Fa. Applied Biosystems. Die Analyse der Phenylthiohydantoin-Aminosäuren erfolgte auf dem online gekoppelten 120A PTH Analyser vom gleichen Hersteller.

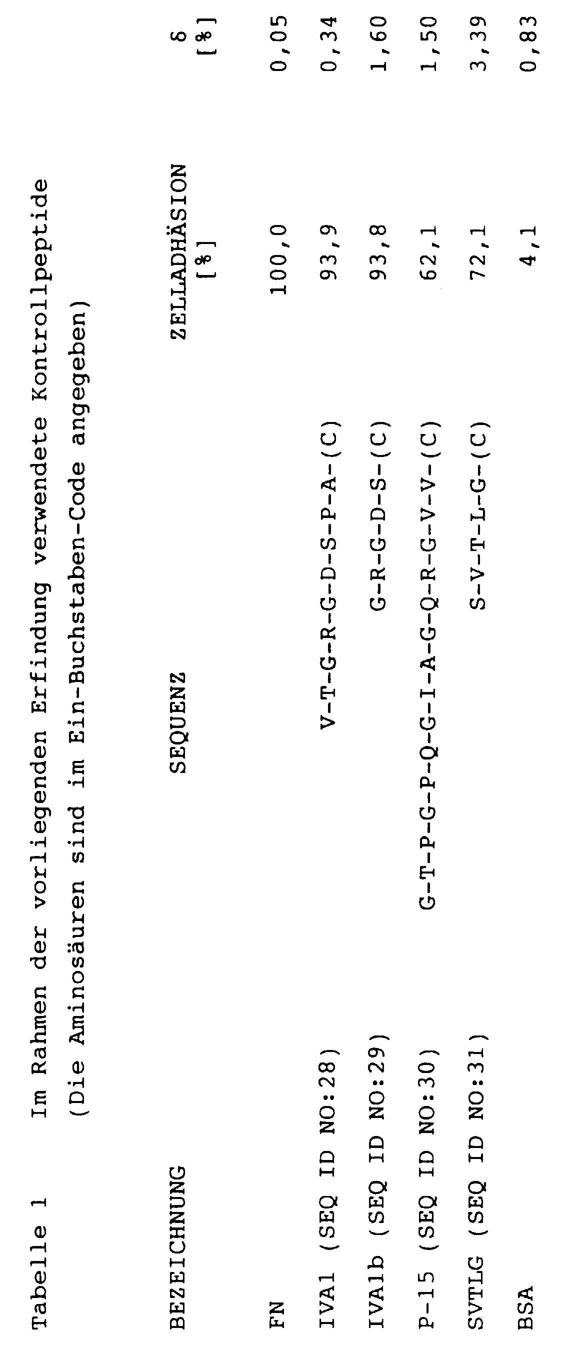

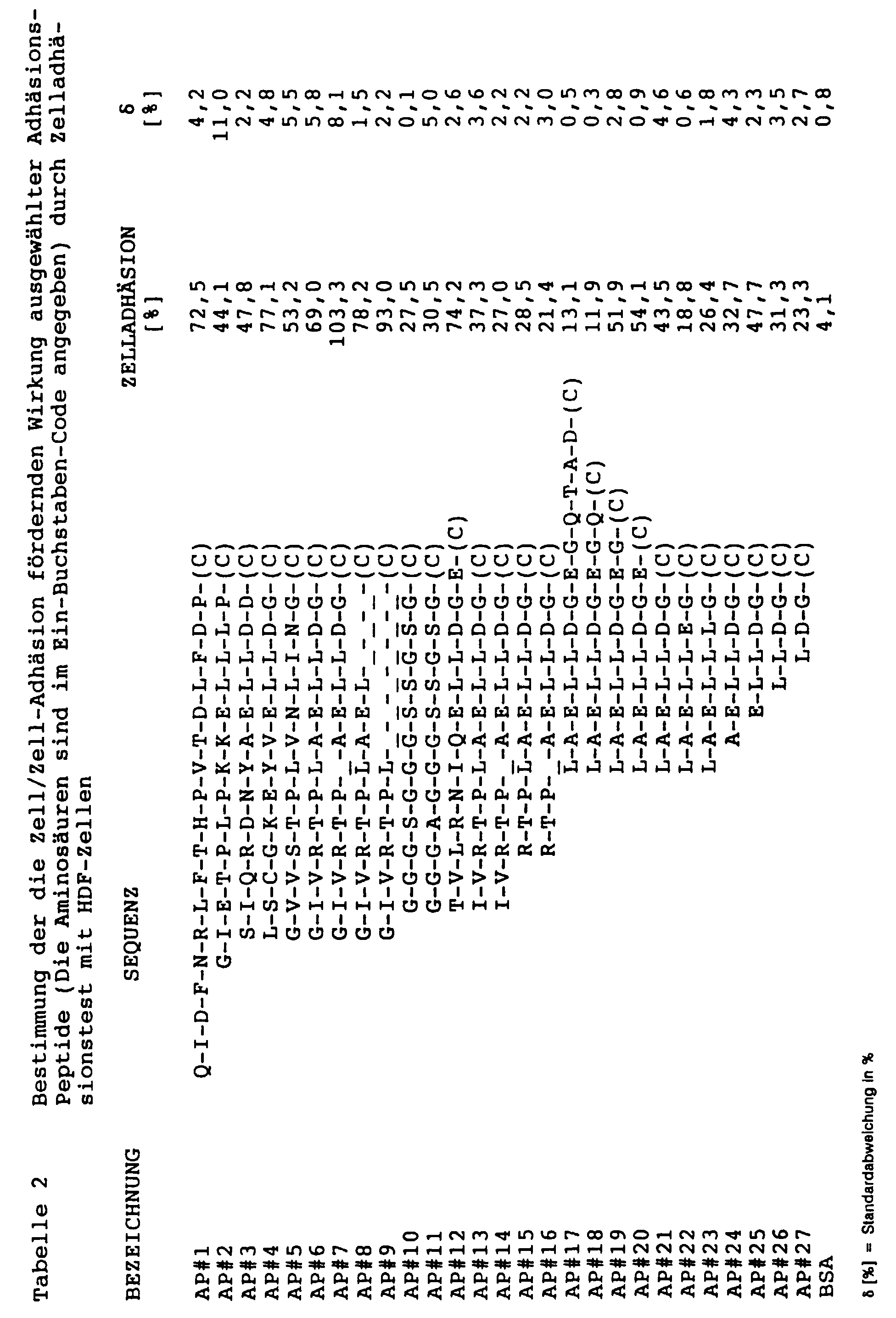

### 2. Kopplung der Peptide an Rinderserumalbumin (BSA) als Carrierprotein

Die mit einem C-terminalen Cystein versehenen Peptide wurden über die reaktive Maleimid-Gruppe des mit dem bifunktionellen Reagenz Sulfosuccinimidyl-4-(N-maleimidomethyl)-cyclohexan-1-carboxylat (Sulfo-SMCC) aktivierten BSA gekoppelt. Dazu wurden die Peptide zur Entferung etwaig vorhandener interpeptidischer Disulfidbindungen zunächst mit Reductacryl® (PIERCE) reduziert. Pro µmol Peptid (einfacher Überschuß) wurden 2,2 mg aktiviertes BSA (PIERCE) verwendet. Die Kopplungsdauer betrug 16 Stunden bei 4° C sowie 2 Stunden bei Raumtemperatur (RT). Nicht gekoppeltes Peptid wurde von dem Konjugat mittels Mikrosep Microconcentrators (Filtron Technology Corp.) mit einem Ausschlußvolumen von 10 KD entfernt.

### 3. Bindung der Testsubstanzen an Polystyrol

Je 50 µl der Peptid-BSA Konjugate bzw. der Peptide wurden in unterschiedlichen Konzentrationen in die Vertiefungen einer 96 Well Mikrotiterplatte mit Flachboden (DYNATECH) gegeben. Nach dem Eintrocknen für 2 Stunden bei 37° C wird ungebundenes Peptid-BSA Konjugat bzw. Peptid durch mehrmaliges Waschen mit phosphatgepufferter Kochsalzlösung (PBS) entfernt. Zur Verhinderung unspezifischer Anhaftung der Zellen an Polystyrol wurden alle Wells anschließend für 30 min. bei 37° C mit hitzedenaturiertem BSA (2 mg/ml) abgesättigt. Anschließend wurde erneut mit PBS gewaschen.

Als Positivkontrolle für das Adhäsionsvermögen wurde Rinder-Fibronektin (Paesel & Lorey, 1 mg/ml), als Negativkontrolle hitzedenaturiertes BSA (2 mg/ml) eingesetzt, welches nicht in der Lage ist, die interzelluläre Adhäsion zu beeinflussen.

### 4. Bestimmung der biologischen Aktivität

### 4.1 Zelladhärenztest

Der Zelladhärenztest wurde mit AKR-2B Mausfibroblasten (Shipley et al., Cancer Res. 44 (1984) 710-716) und humanen dermalen Fibroblasten (HDF, Entnahme aus dem Unterarm, 18. Passage) durchgeführt. AKR-2B Zellen wurden in McCoy's Medium (10 % Kälberserum), HDF Zellen wurden in Dulbecco's Modified Eagle Medium (DMEM) in Gegenwart von 10 % fötalem Kälberserum (FCS) und 1 % Streptomycin/Penicillin kultiviert. Für den Adhärenztest wurden die Zellen 2x mit proteinfreiem Hybri-Max Medium (SIGMA) gewaschen und anschließend in Hybri-Max (1 mM Mn²⁺, 0,02 % hitzedenaturiertes BSA) aufgenommen. Damit wurde weitgehend sichergestellt, daß weder Proteinkomponenten aus dem Kulturmedium, noch Serumkomponenten wie beispielsweise Fibronektin den Adhärenztest beeinflussen. In jede zuvor mit Testsubstanz beschickte Vertiefung der Mikrotiterplatte (Peptidkonzentration/Well ca. 35 nmol) wurden 200 µl der in Hybri-Max (1 mM Mn²⁺, 0,02 % hitzedenaturiertes BSA) aufgenommenen Zellsuspension (1,5 x 10⁵ Zellen/Well) pipettiert und 1 Stunde im Brutschrank bei 37° C, 5 % CO₂-Atmosphäre inkubiert. Danach wurde der Überstand abdekantiert und nicht adhärierte Zellen durch Waschen mit PBS entfernt. Die adhärierten Zellen wurden 0,5 min mit Methanol fixiert und zur Quantifizierung der Zellzahl mit dem Hemacolor-Reagenzien Kit der Fa. MERCK angefärbt.

Für einige ausgewählte Konjugate wurden Verdünnungsreihen zur Ermittlung von Bindungskonstanten für AKR-2B-Zellen aufgenommen. Dazu wurden die Peptid-BSA-Konjugate in PBS verdünnt und je 50 µl dieser Verdünnungen in die Wells einer Mikrotiterplatte gegeben. Die Inkubation und alle weiteren Schritte erfolgten nach dem oben geschilderten Verfahren. Nach graphischer Auftragung der Meßwerte kann die Differenz zwischen dem Wert für die maximale Zelladhärenz und dem Leerwert gebildet und die in Tab. 3 angegebenen EC₅₀-Werte als Maß für die halbmaximale Zelladhärenz aus dem Kurvenverlauf abgelesen werden.

### 4.2 Kompetitiver Hemmtest

Über die spezifische Absättigung von Oberflächenrezeptoren (in Fig. 3E schematisch dargestellt) ist es möglich, für lösliche Peptide einen inhibitorischen Effekt auf die Anheftung von Testzellen (AKR-2B) zu messen. In dem hier beschriebenen Experiment wurde ein ausgewähltes Peptid/BSA-Konjugat entsprechend einer Peptidkonzentration von 10 nmol/Well in den Mikrotiterplatten vorgelegt und für 2 Stunden bei 37° C inkubiert. Ungesättigte Bindungsstellen des Kulturgefäßes wurden anschließend mit hitzedenaturierten BSA (2 mg/ml) für 30 Minuten abgesättigt. Die Peptide wurden in Hybri-Max Medium (0,02 % BSA, 1 mM Mn²⁺) gelöst, und der pH-Wert auf einen Wert von 7,2 bis 7,4 eingestellt. Anschließend wurden die Peptidlösungen mit Reductacryl® behandelt. Dieser Schritt kann bei Peptiden, die kein Cystein beinhalten, entfallen. Von diesen Stammlösungen wurden Verdünnungsreihen in Hybri-Max Medium (1 mM Mn²⁺, 0,02 % hitzedenaturiertes BSA) hergestellt und zu den im gleichen Medium aufgenommenen AKR-2B Zellen gegeben. Anschließend wurde für 10 Minuten unter leichtem Schütteln bei 37°C inkubiert. Die derart vorbehandelten Zellen (1,5 x 10⁵/200 µl) wurden danach in die Konjugat-beschichteten Mikrotiterplatten überführt. Die weitere Behandlung der Testplatten erfolgte analog 4.1.

Die inhibitorische Wirkung der Peptide ist als Differenz des 100 %-Wertes (Kontrolle, ohne Peptid im Medium) zu der bei einer Peptidkonzentration von 1 µmol/Well maximal gemessenen Reduktion der Zelladhärenz in Tab. 4 angegeben. Ein Wert von 100% würde eine vollständigen Inhibition, d.h. die maximale Wirkung, bedeuten.

### 4.3 Chemotaxistest

Neben der Wundkontraktion spielt die Epithelialisierung der Wunde durch Einwanderung (Migration) von Zellen aus den Wundrändern eine wichtige Rolle bei der Wundheilung. Einige ausgewählte Peptide wurden in Chemotaxistests auf ihre Fähigkeit untersucht, die Migration von HDF-Zellen zu simulieren. Der Test wurde gemäß Scharffetter-Kochanek et al. (J. Invest. Dermatol. 98 (1992) 3-11) in modifizierten Boyden-Kammern durchgeführt. In die untere Kavität der Boyden-Kammer wurden 210 µl DMEM (Kontrolle) oder das im gleichen Volumen DMEM aufgenommene potentielle Chemoattraktans vorgelegt. Als Positivkontrolle wurden Kollagen I (300 000 g/ml, 100 µg/ml) und humanes Fibronektin (Boehringer, 25 µg/ml) eingesetzt. In die obere Boyden-Kammer (Volumen 810 µl) wurden 2 x 10⁵ Zellen pipettiert. Die Inkubationszeit betrug 4 Stunden bei 37° C. Die Ergebnisse für die untersuchten Peptide sind in Tab. 5 zusammengefaßt, die Werte für die Mediumkontrolle sind von diesen Meßwerten bereits abgezogen.

### 5. Ergebnisse:

### Ergebnisse der Adhärenztests:

Konjugate aus BSA als Trägermolekül und Kontrollpeptiden, die die RGD-Sequenz enthalten (IVA1 und IVA1b) besitzen für HDF-Zellen erwartungsgemäß ähnlich gute adhärenzfördernde Wirkungen wie Fibronektin (Tab. 1). Sowohl das Kollagen-Fragment P-15, als auch die Thrombospondin-Konsensussequenz SVTLG sind im gewählten Konzentrationsbereich etwas geringer wirksam. BSA, an welches kein Peptid gekoppelt wurde, zeigt praktisch keine adhärenzfördernde Wirkung.

In Tab. 2 sind die mit HDF-Zellen im Adhärenztest erhaltenen Werte für die patentgemäß verwendeten Peptide zusammengestellt. Die Sequenzen der Peptide AP#1 bis AP#27 sind als SEQ ID NO:1 bis SEQ ID NO:27 im Sequenzprotokoll wiedergegeben. Um minimal adhäsionswirksame Epitope näher charakterisieren und weiter eingrenzen zu können, wurden ausgehend von einigen der 13 Aminosäuren langen Sequenzmotive aminoterminal schrittweise um eine oder mehrere Aminosäuren verkürzte Peptidvarianten sowie zusätzlich interne Deletionsvarianten und Peptide mit carboxyterminalen Verlängerungen hergestellt und getestet. Aus den Ergebnissen (vgl. Tab. 2) wird deutlich, daß die aminoterminale Deletion der Aminosäure Glycin eine deutliche Reduktion der Wirksamkeit zur Folge hat (vgl. AP#6 und AP#7 gegenüber AP#13 und AP#14). Die Abnahme der Wirksamkeit ist allerdings nicht auf das bloße Fehlen einer oder mehrerer Aminosäuren zurückzuführen, da überraschenderweise die Variante AP#7, die gegenüber AP#6 durch eine interne Deletion der Aminosäure Leucin in Position 7 gekennzeichnet ist, gegenüber AP#6 sogar eine signifikant verbesserte Adhärenzwirksamkeit zeigt. Für die carboxyterminal verkürzten Varianten AP#8 und AP#9, die aminoterminal die Aminosäure Glycin aufweisen, wurde überraschenderweise ebenfalls eine gute Wirksamkeit nachgewiesen, die mit der adhärenzfördernden Wirkung der vollständigen Sequenz AP#6 vergleichbar ist bzw. diese sogar übersteigt. Die um eine Aminosäure kürzere Sequenz AP#9 zeigte im Vergleich zu AP#8 eine bessere Wirksamkeit. Die Sequenzen AP#8 und AP#9 bzw. Fragmente derselben sind daher als Ausgangspunkt zur Entwicklung weiterer, das Haftungsvermögen eukaryontischer Zellen untereinander wirksam beeinflussender Adhäsionspeptide (z.B. durch Variation der Länge und/oder der Aminosäurefolge, durch Hinzufügen bzw. Entfernen einzelner Aminosäuren) geeignet.

Die mit den Sequenzen AP#8 und AP#9 erzielten Ergebnisse bestätigen, daß die aminoterminale Aminosäure Glycin für die Wirksamkeit der Adhäsions-Peptode von besonderer Bedeutung ist, wie bereits einem Vergleich der Ergebnisse für AP#6 und AP#13 bzw. AP#7 und AP#14 entnommen werden kann.

Wie bereits aus Untersuchungen mit den Peptiden AP#7, AP#14, AP#16 und AP#24 hervorgeht, vermittelt die Aminosäure Leucin (Leu) in Position 7 (AS7) keinen die Adhärenz fördernden bzw. vermittelnden Effekt. Aufgrund der vorliegenden Ergebnisse ist daher die Verwendung der Sequenz für Peptide mit einer Länge von mindestens 9 Aminosäuren im Rahmen der vorliegenden Erfindung bevorzugt.

Ein gemeinsames Hauptmerkmal der am meisten bevorzugt verwendeten adhäsionswirksamen Peptide ist das durch die Definitionen der Ansprüche 1 bis 3 festgelegte carboxyterminale Ende der Peptide, wobei die Verwendung der Aminosäure Glycin als endständige Aminosäure (d.h. AS1 = Gly) bevorzugt ist. Als weitere Aminosäuren in dieser Sequenzposition kommen Prolin (vgl. AP#1, AP#2) und Asparaginsäure (vgl. AP#3) in Frage. Als Ausnahme von dieser Definition wurde im Rahmen der vorliegenden Erfindung gefunden, daß eine Verlängerung dieser definierten Aminosäuresequenz am carboxyterminalen Ende der Peptide um bis zu zwei weitere beliebige Aminosäuren, vorzugsweise Glu und/oder Gly, möglich ist, ohne daß die positive Wirkung auf die Zell/Zell-Interaktion wesentlich gemindert wird. Bei geeigneter Wahl der weiteren Aminosäure bzw. der Aminosäurenkombination kann dies sogar eine erhöhte adhärenzfördernde Wirkung zur Folge haben (vgl. AP#19 und AP#20 gegenüber AP#21).

Andere Varianten mit Sequenzverlängerungen am Carboxyterminus sind gegenüber vergleichbaren Peptiden ohne Verlängerung oder gegenüber Varianten mit nur einer zusätzlichen Aminosäure am carboxyterminalen Ende in der Regel deutlich schwächer wirksam. Das als AP#17 bezeichnete Peptid besitzt die gleiche Sequenzlänge wie die Peptide AP#3 bis AP#6, das mit der Sequenz AP#6 gemeinsame Sequenzraster (nämlich AS7 bis AS1) ist jedoch um 6 Aminosäuren in Richtung des Carboxyterminus verschoben. In ähnlicher Weise gibt es zwischen den Peptiden AP#18 und AP#15 trotz gleicher Sequenzlänge nur eine Überlappung von sieben Aminosäuren. Gegenüber dem Vergleichspeptid AP#21, das nur diese sieben carboxyterminalen Aminosäuren (AS7 bis AS1) der Adhäsions-Peptide AP#6 bzw. AP#15 aufweist, und demgegenüber AP#17 und AP#18 um 6 bzw. 3 Aminosäuren über das carboxyterminale Glycin hinaus verlängert sind, besitzen AP#17 und AP#18 nur eine basale adhäsionsfördernde Wirkung. D.h. Sequenzverlängerungen ab einer Länge von 3 Aminosäuren über das erfindungsgemäß mit AS1 bezeichnete carboxyterminale Ende hinaus üben störende Effekte auf die positive Beeinflussung-der Zell/Zell-Adhärenz aus. Der Einfluß carboxyterminaler Sequenzverlängerungen unterhalb einer Länge von 3 Aminosäuren, d.h. über die als AS1 definierte Aminosäure hinaus, ist relativ gering, wobei sich die um bis zu 3 Aminosäuren verlängerten Adhäsionspeptide im Vergleich zu den carboxyterminal mit AS1 endenden Peptiden im Hinblick auf die interzellulären Interaktionen entweder neutral oder eher adhäsionsfördernd verhalten.

Der Abschnitt mit der größten Sequenzhomologie zwischen den Peptiden AP#3-AP#7 und AP#13-AP#25 liegt im carboxyterminalen Bereich, insbesondere in der Sequenz Glu-Leu-Leu-Asp-Gly.

Die Substitution von Asparaginsäure in Position 2 (AS2) durch Glutaminsäure (Säurefunktion bleibt erhalten), oder durch Leucin (vgl. AP#21 gegenüber AP#22 und AP#23 in Tab. 2) führen zu einer etwas verminderten Adhärenzwirksamkeit.

Bemerkenswert ist, daß selbst für das Tripeptid Leu-Asp-Gly (AP#27) noch eine signifikante adhäsionsfördernde Wirkung nachweisbar ist.

Zusammengefaßt ergibt sich aus diesen Untersuchungen, daß verschiedene Faktoren wesentlichen Einfluß auf die Wirkung der patentgemäß verwendeten Peptidsequenzen bei der Zell/Zell-Interaktion haben. So spielen ein aminoterminales Glycin, sowie die carboxyterminale Kernsequenz Glu-Leu-Leu-Asp-Gly (ELLDG, entspricht AP#25) bei Adhäsions-Peptiden mit mehr als 5 und vorzugsweise mit 12 bis 14 Aminosäuren eine wesentliche Rolle, und interne Sequenzvariationen an den verbleibenden Positionen (wie beispielsweise die interne Deletionsvariante AP#7) können gegenüber AP#6 überraschenderweise sogar verbesserte adhäsionsfördernde Eigenschaften haben. Dagegen haben Sequenzverlängerungen am Carboxyterminus vorwiegend inhibitorischen Einfluß.

Weitere, durch Aminosäure-Austausch erhaltene Peptide wurden im Rahmen der vorliegenden Erfindung ebenfalls auf ihre Fähigkeit, das Haftungsvermögen von eukaryontischen Zellen zu modifizieren, untersucht. Die Ergebnisse sind in Tab. 2 dargestellt. Das Sequenzmuster AP#10 besitzt, genau wie das nahezu identische Adhäsions-Peptid AP#11 (gegenüber AP#10 durch den Austausch der Aminosäure AS9 = Ser gegen AS9 = Ala verschieden) signifikante Adhäsions-modifizierende Wirkung. Eine besonders gute Wirksamkeit wurde mit der Sequenz AP#1 gemessen, ein 18 Aminosäuren langes Adhäsions-Peptid, das die carboxyterminale Aminosäuresequenz Asp-Leu-Phe-Asp-Pro (entspr. AS5-AS4-AS3-AS2-AS1) enthält.

Zur Ermittlung von Bindungskonstanten für die patentgemäß verwendeten Peptide wurden für einige ausgewählte Konjugate Dosis-Wirkungskurven auf AKR-2B-Zellen aufgenommen. Die in Tab. 3 angegebenen EC₅₀-Werte als Maß für die halbmaximale Zelladhärenz wurden aus dem jeweiligen Kurvenverlauf (aufgetragen: Zelladhärenz gegen Peptidkonzentration) abgelesen. Niedrige EC₅₀-Werte bedeuten, daß für das Erreichen eines halbmaximalen Effektes nur geringe Substanzmengen eingesetzt werden müssen, die Substanz also sehr wirksam ist.

Gegenüber dem die Aminosäuresequenz "RGD" enthaltenen Kontrollpeptid IVA1 weisen alle anderen getesteten Peptid-/Carrier Konjugate eine wenigstens um den Faktor 30 (AP#6) geringere adhäsionsvermittelnde Wirkung auf. Allerdings sind in diesem System alle patentgemäß verwendeten Peptide etwa um den Faktor 2-3 wirksamer als das Thrombospondin-Kontrollpeptid "SVTLG". Die geringere Wirksamkeit der patentgemäß verwendeten Peptide gegenüber IVA1 ist vermutlich eine Folge der unterschiedlichen Wirkmechanismen. Wie bereits an anderer Stelle erwähnt, wird die Sequenz "RGD" von Zelloberflächenrezeptoren des Integrin-Typs erkannt, während die patentgemäß verwendeten Peptide in ihrer Wirkungsweise dem Bereich Zell-Zell Interaktion zuzuordnen sind, eine funktionelle Aktivität also über einen anderen Typ von Zelloberflächenrezeptoren entfaltet wird. Die erhaltenen Meßwerte sind somit direkt abhängig von der Anzahl funktioneller Rezeptoren für das jeweilige Peptid-Epitop auf den Testzellen. Vermutlich exprimieren AKR-2B Zellen in vitro weit mehr Rezeptoren des Integrin-Typs als Zelladhäsionsmoleküle. Insgesamt wurden für die patentgemäß verwendeten Peptide sehr einheitliche Werte gemessen, die niedrigsten EC₅₀-Werte wurden für die Peptide AP#3, AP#6 und AP#7 erhalten. Selbst mit dem Pentapeptid "ELLDG" wurde eine im Vergleich zur Kontrolle "SVTLG" bessere Bindungskonstante gemessen.

**Tabelle 3**

| Bezeichnung | EC₅₀ [pmol/Well] |
|---|---|
| IVA1 | 0,73 |
| SVTLG | 64,5 |
| AP#1 | 32,9 |
| AP#2 | 39,8 |
| AP#3 | 24,7 |
| AP#4 | 30,0 |
| AP#6 | 21,5 |
| AP#7 | 27,0 |
| AP#8 | 41,7 |
| AP#9 | 50,7 |
| AP#12 | 29,0 |
| AP#25 | 42,0 |

### Ergebnisse des Adhärenz-Inhibitionstests:

Für die in löslicher Form, d.h. in einer nicht an Trägermoleküle gebundenen Form, vorliegenden, patentgemäß verwendeten Peptide sind Adhäsions-inhibitorische Eigenschaften nachweisbar, die durch die spezifische Blockade von Epitopen auf Oberflächenrezeptoren der Testzellen zustande kommen (vgl. Fig. 3E). Für den Nachweis dieser Effekte wurden ausgewählte Peptide auf ihre Fähigkeit getestet, eine kompetitive Hemmung der Adhärenz der Testzellen an vorgelegtes Konjugat aus BSA und dem Peptid AP#2 zu bewirken.

Als interner Standard wurde das Peptid AP#2 ausgewählt. Die adhäsionsfördernden Eigenschaften von BSA/AP#2 Konjugaten liegen im Vergleich zu den meisten anderen patentgemäß verwendeten Peptiden (vgl. Tab. 2) in einem Bereich halbmaximaler Effektivität. Erfahrungsgemäß ist es für den Nachweis inhibitorischer Effekte günstig, im Bereich einer suboptimalen Wirkung zu messen, da sonst schwache inhibitorische Effekte möglicherweise nicht erfaßt werden.

Als Negativkontrolle wurde das die Sequenz Arg-Gly-Asp ("RGD") enthaltende Peptid IVA1 verwendet. Dieses Peptid entfaltet seine Wirkung über Rezeptoren des Integrin-Typs (vgl. Fig. 1-3), d.h. bei diesem Mechanismus handelt es sich um die spezifische Interaktion von Zellen mit Komponenten der ECM (Zell/Matrix-Wechselwirkung). In dem hier untersuchten Fall der Interaktion zwischen Zelladhäsionsmolekülen im Rahmen eines kompetitiven Tests wurde davon ausgegangen, daß das Peptid IVA1 aufgrund der im Vergleich zur Zell/Zell-Interaktion mechanistisch anders gearteten (s.o.) Wechselwirkung keine spezifische Wirkung aufweist. In Tab. 4 sind die Ergebnisse dieser Untersuchung unter Verwendung der Adhäsions-Peptide AP#3, AP#4, AP#6, AP#7, AP#17 und AP#25 zusammengestellt. Erwartungsgemäß zeigt das Peptid AP#2 (Kompetition mit sich selbst) einen hochsignifikanten Hemm-Effekt auf die Anheftung von AKR-2B Zellen. Ähnlich starke Effekte werden aber auch für die Peptide AP#6 und AP#7, und in etwas geringer Effizienz auch für die Peptide AP#3, AP#4 sowie das kurze Peptid AP#25 gemessen. Dies ist ein eindeutiges Indiz dafür, daß die adhäsionsvermittelnde Funktion dieser Peptidsequenzen auf die Wechselwirkung mit einem gemeinsamen Epitop auf einem zellulären Rezeptor zurückzuführen ist. Das Peptid AP#17, das einen Vertreter für carboxyterminal über AS1 hinaus verlängerte Peptide darstellt, und IVA1, das die bei Zell/Matrix-Interaktionen wirksame RGD-Sequenz (Arg-Gly-Asp) aufweist, zeigten in diesem System erwartungsgemäß praktisch keine Wirkung.

**Tabelle 4**

| Untersuchung der inhibitorischen Wirkung ausgewählter Adhäsions-Peptide | |
|---|---|
| Peptid (Konzentration 1µmol/Well) | Inhibition [%] |
| AP#2 (interner Standard) | 51,5 |
| AP#3 | 17 |
| AP#4 | 19,9 |
| AP#6 | 53 |
| AP#7 | 49,2 |
| AP#17 | 5,4 |
| AP#25 | 18,1 |
| IVA1 (Negativkontrolle) | 2,6 |

### Ergebnisse des Chemotaxistests:

Die Ergebnisse des Chemotaxis-Tests sind in Tab. 5 beispielhaft für die Peptide AP#3 und AP#7 dargestellt. Neben der Bedeutung bei Zell/Matrix-Interaktionen ist für eine Reihe von ECM-Komponenten deren chemotaktische Wirkung bekannt. Insbesondere Kollagen und Fibronektin sind wirksame Aktivatoren der Zellmigration (Guo et al., J. Cell Sci. 96 (1990) 197-205). Nach Abzug der Meßwerte für die Mediumkontrolle zeigen beide getesteten Peptide im Vergleich zu Kollagen und Fibronektin (Positivkontrollen) in der eingesetzten Dosierung einen deutlichen Chemotaxis-Effekt für HDF-Zellen. Durch diese Untersuchung konnte nachgewiesen werden, daß die im Rahmen der vorliegenden Erfindung verwendeten Adhäsions-Peptide *in vitro* die Migration humaner Zellen fördern können und damit ein eindeutiges Potential für die aktive Beschleunigung von Vorgängen der Wundheilung besitzen.

**Tabelle 5**

| Chemotaxis-Test mit HDF-Zellen | | | | |
|---|---|---|---|---|
| | Konzentration | 0̸Zellen/Gesichtsfeld | chemotaktische Migration [%] | δ [%] |
| Kollagen | 0,3 nmol/ml | 45,7 | 100,0 | 16,3 |
| Fibronektin | 0,6 nmol/ml | 43,9 | 96,0 | 5,6 |
| AP#3 | 1µmol/ml | 11,1 | 24,3 | 0,2 |
| AP#7 | 1µmol/ml | 10,9 | 23,9 | 14,2 |

### Beschreibung der Abbildungen

Schematische Darstellung der Zell-Zell und Zell-Matrix Interaktionen
Fig. 1) Kontakte der Zelle mit Komponenten der ECM werden überwiegend von Oberfächenrezeptoren des Integrin-Typs vermittelt, hier dargestellt am Beispiel des Epitops Arg-Gly-Asp ("RGD") (A). Interzelluläre Adhäsionskontakte durch Zelladhäsionsmoleküle ("Cell Adhesion Molecules" - CAM's) sind in (B), induzierbare luminale CAM's in (C) dargestellt.
Fig. 2) Hochspezifische, von Zelladhäsionsmolekülen abgeleitete Peptide, die an geeignete Carriermoleküle gekoppelt vorliegen, sind in der Lage, CAM-spezifische zelluläre Interaktionen zu unterstützen (D). In einer geeigneten galenischen Zubereitung kann mit diesen Konjugaten die Förderung von Vorgängen der Zellanheftung, beispielsweise der Anheftrate von Hauttransplantaten, erreicht werden.
Fig. 3) Diese Darstellung verdeutlicht, in welcher Weise freie, d.h. nicht an Carriermoleküle konjugierte Peptide durch spezifische Absättigung von Bindungsstellen an den CAM's zelluläre Adhäsionsvorgänge inhibieren können (E).

### Abkürzungen:

- AP#: Adhärenz-/Adhäsions-Peptid
- AS: Aminosäure
- BSA: Rinderserumalbumin
- δ: Standardabweichung
- CAM: Cell Adhesion Molecule
- DMEM: Dulbecco's Modified Eagle Medium
- EC₅₀: effective concentration (Wert für die halbmaximale Wirksamkeit
- ECM: Extrazellularmatrix
- FCS: fötales Kälberserum
- HDF: Humane Dermale Fibroblasten
- KD: Kilodalton
- KLH: Keyhole Limpet Hemocyanin
- PBS: phosphatgepufferte Kochsalzlösung
- PEG: Polyethylenglykol
- RGD: Arginin-Glycin-Asparaginsäure
- RT: Raumtemperatur
- TFA: Trifluoressigsäure

## Patentansprüche

1. Verwendung eines Peptids, das die Aminosäuresequenz
AS3-AS2-AS1
aufweist, wobei
AS1 Gly, Pro oder Asp ist,
AS2 Asp, Leu, Asn oder Ser ist
AS3 Leu, Ile, Phe oder Gly ist, und
und wobei
AS1 die endständige Aminosäure am carboxyterminalen Ende des Peptids bezeichnet, wobei die Aminosäuresequenz am carboxyterminalen Ende der Peptide um bis zu zwei beliebige Aminosäuren verlängert sein kann und wobei die Aminosäuresequenz aminoterminal an AS3 bis zu einer Kettenlänge von 30 Aminosäuren verlängert sein kann,
ausgenommen Peptide, die am carboxyterminalen Ende die Sequenz Phe-Ser-Pro oder Phe-Asp-Gly enthalten,
zur Herstellung einer pharmazeutischen Zusammensetzung zur Modifikation des Haftungsvermögens eukaryontischer Zellen untereinander.

2. Verwendung nach Anspruch 1, dadurch gekennzeichnet, daß das Peptid die Aminosäuresequenz
AS4-AS3-AS2-AS1
aufweist, wobei
AS4 Leu oder Ser ist.

3. Verwendung nach den Anspüchen 1 und 2, dadurch gekennzeichnet, daß das Peptid die Aminosäuresequenz
AS5-AS4-AS3-AS2-AS1
aufweist, wobei
AS5 Glu, Ser, Asp oder Asn ist.

4. Verwendung nach den Ansprüchen 1 bis 3, dadurch gekennzeichnet, daß die am carboxyterminalen Ende der Peptide gegebenenfalls zusätzlich vorhandenen Aminosäuren jeweils Glu oder Gly sind.

5. Verwendung nach den Ansprüchen 1 bis 4, dadurch gekennzeichnet, daß das Peptid eine Länge von 6 bis 30 Aminosäuren aufweist, wobei die Aminosäure am aminoterminalen Ende des Peptids Gly, Ser, Leu, Ile, Arg oder Thr ist.

6. Verwendung nach Anspruch 5, dadurch gekennzeichnet, daß das Peptid eine Länge von 12 bis 14 Aminosäuren aufweist.

7. Verwendung nach Anspruch 6, dadurch gekennzeichnet, daß die Aminosäure am aminoterminalen Ende des Peptids Gly ist.

8. Verwendung nach den Ansprüchen 1 bis 7, dadurch gekennzeichnet, daß AS3 Leu ist.

9. Verwendung nach den Ansprüchen 3 bis 8, dadurch gekennzeichnet, daß AS5 Glu ist.

10. Verwendung nach Anspruch 1, dadurch gekennzeichnet, daß das Peptid eine Länge von drei Aminosäuren aufweist, wobei die Aminosäurefolge Leu-Asp-Gly (SEQ ID NO: 27) ist.

11. Verwendung nach Anspruch 3, dadurch gekennzeichnet, daß das Peptid die Aminosäurefolge oder aufweist.

12. Verwendung nach Anspruch 3, dadurch gekennzeichnet, daß das Peptid die Aminosäurefolge oder aufweist.

13. Verwendung nach Anspruch 3, dadurch gekennzeichnet, daß das Peptid die Aminosäurefolge aufweist.

14. Verwendung nach Anspruch 3, dadurch gekennzeichnet, daß das Peptid die Aminosäurefolge oder aufweist.

15. Verwendung nach Anspruch 3, dadurch gekennzeichnet, daß das Peptid die Aminosäurefolge aufweist.

16. Verwendung nach Anspruch 3, dadurch gekennzeichnet, daß das Peptid die Aminosäurefolge aufweist.

17. Verwendung nach Anspruch 3, dadurch gekennzeichnet, daß das Peptid die Aminosäurefolge aufweist.

18. Verwendung eines Peptids aus der Gruppe bestehend aus oder oder eines Fragments dieser Peptide zur Herstellung einer pharmazeutischen Zusammensetzung zur Modifikation des Haftungsvermögens eukaryontischer Zellen untereinander.

19. Verwendung nach einem der Ansprüche 1 bis 18, dadurch gekennzeichnet, daß das Peptid an einen Träger gebunden ist.

20. Verwendung nach Anspruch 19, dadurch gekennzeichnet, daß der Träger BSA, KLH, Kollagen, Polylysin, ein Immunglobulin oder ein Polysaccharid ist.

21. Verwendung nach Anspruch 20, dadurch gekennzeichnet, daß das Polysaccharid Hyaluronsäure ist.

22. Verwendung nach den Ansprüchen 19 bis 21, dadurch gekennzeichnet, daß das Peptid an seinem carboxyterminalen Ende über einen Cysteinrest an den Träger gebunden ist.

23. Verwendung nach den Ansprüchen 19 bis 22 zur Förderung der Zell/Zell-Adhäsion eukaryontischer Zellen, wobei mindestens zwei Peptide an eine einzelne Trägereinheit gebunden sind.

24. Verwendung nach Anspruch 23, dadurch gekennzeichnet, daß die pharmazeutische Zusammensetzung die Anwachsrate von Hauttransplantaten oder Organtransplantaten steigert.

25. Verwendung nach Anspruch 23, dadurch gekennzeichnet, daß die pharmazeutische Zusammensetzung die Wundheilung fördert.

26. Verwendung nach Anspruch 23, wobei die eukaryontischen Zellen Endothelialzellen sind.

27. Verwendung nach Anspruch 26, wobei die Endothelialzellen gefäßauskleidende Endothelialzellen in künstlichen Blutgefäßen sind.

28. Verwendung nach Anspruch 23, wobei die eukaryontischen Zellen humane dermale und/oder epidermale Zellen sind.

29. Verwendung nach Anspruch 28 zur Fixierung von Hauttransplantaten.

30. Verwendung der Peptide nach den Ansprüchen 1 bis 18 zur Inhibition oder Reduktion der Zell/Zell-Adhäsion eukaryontischer Zellen.

31. Verwendung nach den Ansprüchen 19 bis 22 zur Inhibition oder Reduktion der Zell/Zell-Adhäsion eukaryontischer Zellen, wobei jeweils ein einzelnes Peptid an eine einzelne Trägereinheit gebunden ist.

32. Verwendung nach Anspruch 30 oder 31, dadurch gekennzeichnet, daß die pharmazeutische Zusammensetzung zur Thromboseprophylaxe und/oder Arterioskleroseprophylaxe ist.

33. Verwendung nach Anspruch 30 oder 31, dadurch gekennzeichnet, daß die pharmazeutische Zusammensetzung zur Unterdrückung der Metastasierung von Krebszellen ist.

34. Verwendung nach Anspruch 30 oder 31, dadurch gekennzeichnet, daß die pharmazeutische Zusammensetzung zur Entzündungshemmung ist.

35. Verwendung nach den Ansprüchen 1 bis 18 zur Inhibition oder Reduktion der Zell/Zell-Adhäsion eukaryontischer Zellen in vitro.

36. Verwendung nach den Ansprüchen 1 bis 22 als chemotaktischer Attraktor in vitro.

37. Verwendung nach den Ansprüchen 1 bis 22 zur qualitativen und/oder quantitativen Bestimmung der Zell/Zell-Adhäsion eukaryontischer Zellen in vitro.

38. Verwendung nach den Ansprüchen 23 und 26 bis 29 zur Förderung der Zell/Zell-Adhäsion eukaryontischer Zellen in vitro.

39. Verwendung nach Anspruch 31 zur Inhibition der Zell/Zell-Adhäsion eukaryontischer Zellen in vitro.

## Claims

1. Use of a peptide which has the amino acid sequence
AA3-AA2-AA1,
where
AA1 is Gly, Pro or Asp,
AA2 is Asp, Leu, Asn or Ser,
AA3 is Leu, Ile, Phe or Gly,
and where
AA1 indicates the terminal amino acid at the carboxy-terminal end of the peptide, it being possible for the amino acid sequence on the carboxy-terminal end of the peptides to be extended by up to two amino acids as desired, and it being possible for the amino acid sequence at the amino terminus of AA3 to be extended up to a chain length of 30 amino acids,
with the exception of peptides which contain the sequence Phe-Ser-Pro or Phe-Asp-Gly at the carboxy-terminal end,
for the manufacture of a pharmaceutical composition for modifying the adhesion capacity of eukaryotic cells between each other.

2. Use according to Claim 1, characterized in that the peptide has the amino acid sequence
AA4-AA3-AA2-AA1,
where
AA4 is Leu or Ser.

3. Use according to Claims 1 and 2, characterized in that the peptide has the amino acid sequence
AA5-AA4-AA3-AA2-AA1,
where
AA5 is Glu, Ser, Asp or Asn.

4. Use according to Claims 1 to 3, wherein the amino acids which are optionally additionally present at the carboxy-terminal end of the peptides are in each case Glu or Gly.

5. Use according to Claims 1 to 4, wherein the peptide has a length of 6 to 30 amino acids, in which the amino acid at the amino-terminal end of the peptide is Gly, Ser, Leu, Ile, Arg or Thr.

6. Use according to Claim 5, characterized in that the peptide has a length of 12 to 14 amino acids.

7. Use according to Claim 6, characterized in that the amino acid at the amino-terminal end of the peptide is Gly.

8. Use according to Claims 1 to 7, wherein AA3 is Leu.

9. Use according to Claims 3 to 8, wherein AA5 is Glu.

10. Use according to Claim 1, characterized in that the peptide has a length of three amino acids, the amino acid sequence being Leu-Asp-Gly (SEQ ID NO: 27).

11. Use according to Claim 3, characterized in that the peptide has the amino acid sequence or

12. Use according to Claim 3, characterized in that the peptide has the amino acid sequence or

13. Use according to Claim 3, characterized in that the peptide has the amino acid sequence

14. Use according to Claim 3, characterized in that the peptide has the amino acid sequence or

15. Use according to Claim 3, characterized in that the peptide has the amino acid sequence

16. Use according to Claim 3, characterized in that the peptide has the amino acid sequence

17. Use according to Claim 3, characterized in that the peptide has the amino acid sequence

18. Use of a peptide from the group consisting of or or of a fragment of these peptides for the manufacture of a pharmaceutical composition for modifying the adhesion capacity of eukaryotic cells between each other.

19. Use according to Claims 1 to 18, characterized in that the peptide is bound to a support.

20. Use according to Claim 19, characterized in that the support is BSA, KLH, collagen, polylysine, an immunoglobulin or a polysaccharide.

21. Use according to Claim 20, characterized in that the polysaccharide is hyaluronic acid.

22. Use according to Claims 19 to 21, characterized in that the peptide is bound to the support with its carboxy-terminal end via a cysteine residue.

23. Use according to Claims 19 to 22 for promoting the cell/cell adhesion of eukaryotic cells, in which at least two peptides are bound to a single support unit.

24. Use according to Claim 23, characterized in that the pharmaceutical composition increases the growth rate of skin grafts or organ transplants.

25. Use according to Claim 23, characterized in that the pharmaceutical composition promotes wound healing.

26. Use according to Claim 23, in which the eukaryotic cells are endothelial cells.

27. Use according to Claim 26, in which the endothelial cells are vessel-lining endothelial cells in artificial blood vessels.

28. Use according to Claim 23, in which the eukaryotic cells are human dermal and/or epidermal cells.

29. Use according to Claim 28 for fixing skin grafts.

30. Use of the peptides according to Claims 1 to 18 for inhibiting or reducing the cell/cell adhesion of eukaryotic cells.

31. Use according to Claims 19 to 22 for inhibiting or reducing the cell/cell adhesion of eukaryotic cells, in which a single peptide is bound to a single support unit in each case.

32. Use according to Claim 30 or 31, characterized in that the pharmaceutical composition is for the prophylaxis of thrombosis and/or for the prophylaxis of arteriosclerosis.

33. Use according to Claim 30 or 31, characterized in that the pharmaceutical composition is for suppressing the metastasis of cancer cells.

34. Use according to Claim 30 or 31, characterized in that the pharmaceutical composition is for the inhibition of inflammation.

35. Use according to Claims 1 to 18 for inhibiting or reducing the cell/cell adhesion of eukaryotic cells in vitro.

36. Use according to Claims 1 to 22, as a chemotactic attractor in vitro.

37. Use according to Claims 1 to 22 for the qualitative and/or quantitative determination of the cell/cell adhesion of eukaryotic cells in vitro.

38. Use according to Claims 23 and 26 to 29 for promoting the cell/cell adhesion of eukaryotic cells in vitro.

39. Use according to Claim 31 for inhibiting the cell/cell adhesion of eukaryotic cells in vitro.

## Revendications

1. Utilisation d'un peptide qui présente la séquence d'acides aminés
AS3-AS2-AS1
AS1 représente Gly Pro ou Asp,
AS2 représente Asp, Leu, Asn ou Ser,
AS3 représente Leu, Ile, Phe ou Gly, et
où
AS1 représente l'acide aminé terminal à l'extrémité C-terminale du peptide, la séquence d'acides aminés à l'extrémité C-terminale des peptides pouvant être allongée de jusqu'à deux acides aminés arbitraires et la séquence d'acides aminés N-terminale sur AS3 peut être allongée jusqu'à une longueur de chaîne de 30 acides aminés,
à l'exception de peptides qui présentent à l'extrémité C-terminale la séquence Phe-Ser-Pro ou Phe-Asp-Gly,
pour la préparation d'une composition pharmaceutique pour modifier le pouvoir d'adhésion de cellules eucaryotes.

2. Utilisation selon la revendication 1, caractérisée en ce que le peptide présente la séquence d'acides aminés
AS4-AS3-AS2-AS1
AS4 représentant Leu ou Ser.

3. Utilisation selon les revendications 1 et 2, caractérisée en ce que le peptide présente la séquence d'acides aminés
AS5-AS4-AS3-AS2-AS1
AS5 représentant Glu, Ser, Asp ou Asn.

4. Utilisation selon les revendications 1 à 3, caractérisée en ce que les acides aminés éventuellement présents de plus à l'extrémité C-terminale des peptides sont à chaque fois Glu ou Gly.

5. Utilisation selon les revendications 1 à 4, caractérisée en ce que le peptide présente une longueur de 6 à 30 acides aminés, l'acide aminé à l'extrémité N-terminale du peptide étant Gly, Ser, Leu, Ile, Arg ou Thr.

6. Utilisation selon la revendication 5, caractérisée en ce que le peptide présente une longueur de 12 à 14 acides aminés.

7. Utilisation selon la revendication 6, caractérisée en ce que l'acide aminé à l'extrémité aminoterminale du peptide est Gly.

8. Utilisation selon les revendications 1 à 7, caractérisée en ce que AS3 est Leu.

9. Utilisation selon les revendications 3 à 8, caractérisée en ce que AS5 est Glu.

10. Utilisation selon la revendication 1, caractérisée en ce que le peptide présente une longueur de 3 acides aminés, l'enchaînement des acides aminés étant

11. Utilisation selon la revendication 3, caractérisée en ce que le peptide présente l'enchaînement d'acides aminés ou

12. Utilisation selon la revendication 3, caractérisée en ce que le peptide présente l'enchaînement d'acides aminés ou

13. Utilisation selon la revendication 3, caractérisée en ce que le peptide présente l'enchaînement d'acides aminés

14. Utilisation selon la revendication 3, caractérisée en ce que le peptide présente l'enchaînement d'acides aminés ou

15. Utilisation selon la revendication 3, caractérisée en ce que le peptide présente l'enchaînement d'acides aminés

16. Utilisation selon la revendication 3, caractérisée en ce que le peptide présente l'enchaînement d'acides aminés

17. Utilisation selon la revendication 3, caractérisée en ce que le peptide présente l'enchaînement d'acides aminés

18. Utilisation d'un des peptides pris dans le groupe constitué par 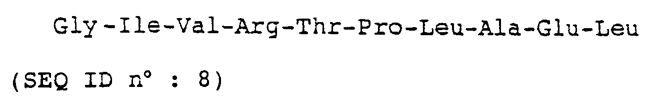 ou ou d'un fragment de ces peptides pour la préparation d'une composition pharmaceutique pour modifier le pouvoir d'adhésion de cellules eucaryotes entre elles.

19. Utilisation selon l'une des revendications 1 à 18, caractérisée en ce que le peptide est lié à un support.

20. Utilisation selon la revendication 19, caractérisée en ce que le support est l'ASB, le KLH, le collagène, la polylysine, une immunoglobuline ou un polysaccharide.

21. Utilisation selon la revendication 20, caractérisée ne ce que le polysaccharide est l'acide hyaluronique.

22. Utilisation selon les revendications 19 à 21 caractérisée en ce que le peptide est lié au support à son extrémité C-terminale par l'intermédiaire d'un reste cystéine.

23. Utilisation selon les revendications 19 à 22 pour la stimulation de l'adhésion cellule/cellule de cellules eucaryotes, au moins deux peptides étant liés à une seule unité de support.

24. Utilisation selon la revendication 23, caractérisée en ce que la composition pharmaceutique augmente le taux de prise de greffons de peau ou de greffes d'organes.

25. Utilisation selon la revendication 23, caractérisée en ce que la composition pharmaceutique favorise la cicatrisation.

26. Utilisation selon la revendication 23, où les cellules eucaryotes sont des cellules endothéliales.

27. Utilisation selon la revendication 26, où les cellules endothéliales sont des cellules endothéliales qui forment le revêtement des vaisseaux sanguins artificiels.

28. Utilisation selon la revendication 23, où les cellules eucaryotes sont des cellules dermiques et/ou épidermiques humaines.

29. Utilisation selon la revendication 28 pour la fixation de greffons de peau.

30. Utilisation selon les revendications 1 à 18 pour l'inhibition ou la réduction de l'adhésion cellule/cellule de cellules eucaryotes.

31. Utilisation selon les revendications 19 à 22 pour l'inhibition ou la réduction de l'adhésion cellule/cellule de cellules eucaryotes, à chaque fois un seul petide étant lié à une seule unité de support.

32. Utilisation selon la revendication 30 ou 31, caractérisée en ce que la composition pharmaceutique est destinée à la prévention de thromboses et/ou d'artériosclérose.

33. Utilisation selon la revendication 30 ou 31, caractérisée en ce que la composition pharmaceutique est destinée à supprimer les métastases de cellules cancéreuses.

34. Utilisation selon la revendication 30 ou 31 caractérisée en la composition pharmaceutique est prévue pour l'inhibition de processus inflammatoires.

35. Utilisation selon les revendications 1 à 18 pour l'inhibition ou la réduction de l'adhésion in vitro cellule/cellule de cellules eucaryotes.

36. Utilisation selon les revendications 1 à 22 en tant que qu'attracteur chimiotactique in vitro.

37. Utilisation selon les revendications 1 à 22 pour la détermination qualitative et/ou quantitative de l'adhésion cellule/cellule de cellules eucaryotes in vitro.

38. Utilisation selon les revendications 23 et 26 à 29 pour la stimulation de l'adhésion cellule/cellule de cellules eucaryotes in vitro.

39. Utilisation selon la revendication 31 pour l'inhibition de l'adhésion cellule/cellule de cellules eucaryotes in vitro.


### SEQUENZPROTOKOLL

(1) ALLGEMEINE ANGABEN:
   (i) ANMELDER:
      (A) NAME: Beiersdorf AG
      (B) STRASSE: Unnastr. 48
      (C) ORT: Hamburg
      (E) LAND: Deutschland
      (F) POSTLEITZAHL: 20245
   (ii) BEZEICHNUNG DER ERFINDUNG: Zelladhaesions-Peptide zur Modifikation des Haftungsvermoegens eukaryontischer Zellen untereinander
   (iii) ANZAHL DER SEQUENZEN: 35
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 18 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..18
      (D) SONSTIGE ANGABEN:/note= "AP#1"
   (xi) SEQUENZBESCHREIBUNG: SEQ ID NO: 1:
   Gln Ile Asp Phe Asn Arg Leu Phe Thr His Pro Val Thr Asp Leu Phe
   1               5                   10                  15
   Asp Pro
(2) ANGABEN ZU SEQ ID NO: 2:
   (i) SEQUENZKENNZEICHEN:
      (A) LÄNGE: 14 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..14
      (D) SONSTIGE ANGABEN:/note= "AP#2"
   (xi) SEQUENZBESCHREIBUNG: SEQ ID NO: 2:
   Gly Ile Glu Thr Pro Leu Pro Lys Lys Glu Leu Leu Leu Pro
   1               5                   10
(2) ANGABEN ZU SEQ ID NO: 3:
   (i) SEQUENZKENNZEICHEN:
      (A) LÄNGE: 13 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..13
      (D) SONSTIGE ANGABEN:/note= "AP#3"
   (xi) SEQUENZBESCHREIBUNG: SEQ ID NO: 3:
   Ser Ile Gln Arg Asp Asn Tyr Ala Glu Leu Leu Asp Asp
   1               5                   10
(2) ANGABEN ZU SEQ ID NO: 4:
   (i) SEQUENZKENNZEICHEN:
      (A) LÄNGE: 13 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..13
      (D) SONSTIGE ANGABEN:/note= "AP#4"
   (xi) SEQUENZBESCHREIBUNG: SEQ ID NO: 4:
   Leu Ser Cys Gly Lys Glu Tyr Val Glu Leu Leu Asp Gly
   1               5                   10
(2) ANGABEN ZU SEQ ID NO: 5:
   (i) SEQUENZKENNZEICHEN:
      (A) LÄNGE: 13 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..13
      (D) SONSTIGE ANGABEN:/note= "AP#5"
   (xi) SEQUENZBESCHREIBUNG: SEQ ID NO: 5:
   Gly Val Val Ser Thr Pro Leu Val Asn Leu Ile Asn Gly
   1               5                   10
(2) ANGABEN ZU SEQ ID NO: 6:
   (i) SEQUENZKENNZEICHEN:
      (A) LÄNGE: 13 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..13
      (D) SONSTIGE ANGABEN:/note= "AP#6"
   (xi) SEQUENZBESCHREIBUNG: SEQ ID NO: 6:
   Gly Ile Val Arg Thr Pro Leu Ala Glu Leu Leu Asp Gly
   1               5                   10
(2) ANGABEN ZU SEQ ID NO: 7:
   (i) SEQUENZKENNZEICHEN:
      (A) LÄNGE: 12 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..12
      (D) SONSTIGE ANGABEN:/note= "AP#7"
   (xi) SEQUENZBESCHREIBUNG: SEQ ID NO: 7:
   Gly Ile Val Arg Thr Pro Ala Glu Leu Leu Asp Gly
   1               5                   10
(2) ANGABEN ZU SEQ ID NO: 8:
   (i) SEQUENZKENNZEICHEN:
      (A) LÄNGE: 10 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..10
      (D) SONSTIGE ANGABEN:/note= "AP#8"
   (xi) SEQUENZBESCHREIBUNG: SEQ ID NO: 8:
   Gly Ile Val Arg Thr Pro Leu Ala Glu Leu
   1               5                   10
(2) ANGABEN ZU SEQ ID NO: 9:
   (i) SEQUENZKENNZEICHEN:
      (A) LÄNGE: 7 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..7
      (D) SONSTIGE ANGABEN:/note= "AP#9"
   (xi) SEQUENZBESCHREIBUNG: SEQ ID NO: 9:
   Gly Ile Val Arg Thr Pro Leu
   1               5
(2) ANGABEN ZU SEQ ID NO: 10:
   (i) SEQUENZKENNZEICHEN:
      (A) LÄNGE: 12 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..12
      (D) SONSTIGE ANGABEN:/note= "AP#10"
   (xi) SEQUENZBESCHREIBUNG: SEQ ID NO: 10:
   Gly Gly Gly Ser Gly Gly Gly Ser Ser Gly Ser Gly
   1               5                   10
(2) ANGABEN ZU SEQ ID NO: 11:
   (i) SEQUENZKENNZEICHEN:
      (A) LÄNGE: 12 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..12
      (D) SONSTIGE ANGABEN:/note= "AP#11"
   (xi) SEQUENZBESCHREIBUNG: SEQ ID NO: 11:
   Gly Gly Gly Ala Gly Gly Gly Ser Ser Gly Ser Gly
   1               5                   10
(2) ANGABEN ZU SEQ ID NO: 12:
   (i) SEQUENZKENNZEICHEN:
      (A) LÄNGE: 13 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..13
      (D) SONSTIGE ANGABEN:/note= "AP#12"
   (xi) SEQUENZBESCHREIBUNG: SEQ ID NO: 12:
   Thr Val Leu Arg Asn Ile Gln Glu Leu Leu Asp Gly Glu
   1               5                   10
(2) ANGABEN ZU SEQ ID NO: 13:
   (i) SEQUENZKENNZEICHEN:
      (A) LÄNGE: 12 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..12
      (D) SONSTIGE ANGABEN:/note= "AP#13"
   (xi) SEQUENZBESCHREIBUNG: SEQ ID NO: 13:
   Ile Val Arg Thr Pro Leu Ala Glu Leu Leu Asp Gly
   1               5                   10
(2) ANGABEN ZU SEQ ID NO: 14:
   (i) SEQUENZKENNZEICHEN:
      (A) LÄNGE: 11 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..11
      (D) SONSTIGE ANGABEN:/note= "AP#14"
   (xi) SEQUENZBESCHREIBUNG: SEQ ID NO: 14:
   Ile Val Arg Thr Pro Ala Glu Leu Leu Asp Gly
   1               5                   10
(2) ANGABEN ZU SEQ ID NO: 15:
   (i) SEQUENZKENNZEICHEN:
      (A) LÄNGE: 10 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..10
      (D) SONSTIGE ANGABEN:/note= "AP#15"
   (xi) SEQUENZBESCHREIBUNG: SEQ ID NO: 15:
   Arg Thr Pro Leu Ala Glu Leu Leu Asp Gly
   1               5                   10
(2) ANGABEN ZU SEQ ID NO: 16:
   (i) SEQUENZKENNZEICHEN:
      (A) LÄNGE: 9 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..9
      (D) SONSTIGE ANGABEN:/note= "AP#16"
   (xi) SEQUENZBESCHREIBUNG: SEQ ID NO: 16:
   Arg Thr Pro Ala Glu Leu Leu Asp Gly
   1               5
(2) ANGABEN ZU SEQ ID NO: 17:
   (i) SEQUENZKENNZEICHEN:
      (A) LÄNGE: 13 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..13
      (D) SONSTIGE ANGABEN:/note= "AP#17"
   (xi) SEQUENZBESCHREIBUNG: SEQ ID NO: 17:
   Leu Ala Glu Leu Leu Asp Gly Glu Gly Gln Thr Ala Asp
   1               5                   10
(2) ANGABEN ZU SEQ ID NO: 18:
   (i) SEQUENZKENNZEICHEN:
      (A) LÄNGE: 10 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..10
      (D) SONSTIGE ANGABEN:/note= "AP#18"
   (xi) SEQUENZBESCHREIBUNG: SEQ ID NO: 18:
   Leu Ala Glu Leu Leu Asp Gly Glu Gly Gln
   1               5                   10
(2) ANGABEN ZU SEQ ID NO: 19:
   (i) SEQUENZKENNZEICHEN:
      (A) LÄNGE: 9 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..9
      (D) SONSTIGE ANGABEN:/note= "AP#19"
   (xi) SEQUENZBESCHREIBUNG: SEQ ID NO: 19:
   Leu Ala Glu Leu Leu Asp Gly Glu Gly
   1               5
(2) ANGABEN ZU SEQ ID NO: 20:
   (i) SEQUENZKENNZEICHEN:
      (A) LÄNGE: 8 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..8
      (D) SONSTIGE ANGABEN:/note= "AP#20"
   (xi) SEQUENZBESCHREIBUNG: SEQ ID NO: 20:
   Leu Ala Glu Leu Leu Asp Gly Glu
   1               5
(2) ANGABEN ZU SEQ ID NO: 21:
   (i) SEQUENZKENNZEICHEN:
      (A) LÄNGE: 7 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..7
      (D) SONSTIGE ANGABEN:/note= "AP#21"
   (xi) SEQUENZBESCHREIBUNG: SEQ ID NO: 21:
   Leu Ala Glu Leu Leu Asp Gly
   1               5
(2) ANGABEN ZU SEQ ID NO: 22:
   (i) SEQUENZKENNZEICHEN:
      (A) LÄNGE: 7 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..7
      (D) SONSTIGE ANGABEN:/note= "AP#22"
   (xi) SEQUENZBESCHREIBUNG: SEQ ID NO: 22:
   Leu Ala Glu Leu Leu Glu Gly
   1               5
(2) ANGABEN ZU SEQ ID NO: 23:
   (i) SEQUENZKENNZEICHEN:
      (A) LÄNGE: 7 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..7
      (D) SONSTIGE ANGABEN:/note= "AP#23"
   (xi) SEQUENZBESCHREIBUNG: SEQ ID NO: 23:
   Leu Ala Glu Leu Leu Leu Gly
   1               5
(2) ANGABEN ZU SEQ ID NO: 24:
   (i) SEQUENZKENNZEICHEN:
      (A) LÄNGE: 6 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..6
      (D) SONSTIGE ANGABEN:/note= "AP#24"
   (xi) SEQUENZBESCHREIBUNG: SEQ ID NO: 24:
   Ala Glu Leu Leu Asp Gly
   1               5
(2) ANGABEN ZU SEQ ID NO: 25:
   (i) SEQUENZKENNZEICHEN:
      (A) LÄNGE: 5 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..5
      (D) SONSTIGE ANGABEN:/note= "AP#25"
   (xi) SEQUENZBESCHREIBUNG: SEQ ID NO: 25:
   Glu Leu Leu Asp Gly
   1               5
(2) ANGABEN ZU SEQ ID NO: 26:
   (i) SEQUENZKENNZEICHEN:
      (A) LÄNGE: 4 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..4
      (D) SONSTIGE ANGABEN:/note- "AP#26"
   (xi) SEQUENZBESCHREIBUNG: SEQ ID NO: 26:
   Leu Leu Asp Gly
   1
(2) ANGABEN ZU SEQ ID NO: 27:
   (i) SEQUENZKENNZEICHEN:
      (A) LÄNGE: 3 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..3
      (D) SONSTIGE ANGABEN:/note= "AP#27"
   (xi) SEQUENZBESCHREIBUNG: SEQ ID NO: 27:
   Leu Asp Gly
   1
(2) ANGABEN ZU SEQ ID NO: 28:
   (i) SEQUENZKENNZEICHEN:
      (A) LÄNGE: 9 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..9
      (D) SONSTIGE ANGABEN:/note= "IVA1"
   (xi) SEQUENZBESCHREIBUNG: SEQ ID NO: 28:
   Val Thr Gly Arg Gly Asp Ser Pro Ala
   1               5
(2) ANGABEN ZU SEQ ID NO: 29:
   (i) SEQUENZKENNZEICHEN:
      (A) LÄNGE: 5 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..5
      (D) SONSTIGE ANGABEN:/note= "IVA1b"
   (xi) SEQUENZBESCHREIBUNG: SEQ ID NO: 29:
   Gly Arg Gly Asp Ser
   1               5
(2) ANGABEN ZU SEQ ID NO: 30:
   (i) SEQUENZKENNZEICHEN:
      (A) LÄNGE: 15 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..15
      (D) SONSTIGE ANGABEN:/note= "P-15"
   (xi) SEQUENZBESCHREIBUNG: SEQ ID NO: 30:
   Gly Thr Pro Gly Pro Gln Gly Ile Ala Gly Gln Arg Gly Val Val
   1 5 10 15
(2) ANGABEN ZU SEQ ID NO: 31:
   (i) SEQUENZKENNZEICHEN:
      (A) LÄNGE: 5 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..5
      (D) SONSTIGE ANGABEN:/note= "SVTLG"
   (xi) SEQUENZBESCHREIBUNG: SEQ ID NO: 31:
   Ser Val Thr Leu Gly
   1 5
(2) ANGABEN ZU SEQ ID NO: 32:
   (i) SEQUENZKENNZEICHEN:
      (A) LÄNGE: 6 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..6
      (D) SONSTIGE ANGABEN:/note= "NH2-Terminus"
   (xi) SEQUENZBESCHREIBUNG: SEQ ID NO: 32:
   Gly Ile Val Arg Thr Pro
   1 5
(2) ANGABEN ZU SEQ ID NO: 33:
   (i) SEQUENZKENNZEICHEN:
      (A) LÄNGE: 5 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..5
      (D) SONSTIGE ANGABEN:/note= "Die Aminoaeuren koennen folgende Bedeutung haben: Pos. 1 (=AS3): Leu, Ile, Phe od. Gly / Pos. 2 (=AS2): Asp, Leu, Asn od. Ser / Pos. 3 (=AS1): Gly, Pro od. Asp / Pos. 4 und 5 (ASX, ASY): beliebig"
   (xi) SEQUENZBESCHREIBUNG: SEQ ID NO: 33:
   Xaa Xaa Xaa Xaa Xaa
   1               5
(2) ANGABEN ZU SEQ ID NO: 34:
   (i) SEQUENZKENNZEICHEN:
      (A) LÄNGE: 6 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..6
      (D) SONSTIGE ANGABEN:/note= "Die Aminoaeuren koennen folgende Bedeutung haben: Pos. 1 (=AS4): Leu od. Ser / Bedeutung der anderen Aminosaeuren wie in SEQ.ID Nr. 33"
   (xi) SEQUENZBESCHREIBUNG: SEQ ID NO: 34:
   Xaa Xaa Xaa Xaa Xaa Xaa
   1               5
(2) ANGABEN ZU SEQ ID NO: 35:
   (i) SEQUENZKENNZEICHEN:
      (A) LÄNGE: 7 Aminosäuren
      (B) ART: Aminosäure
      (C) STRANGFORM:
      (D) TOPOLOGIE: linear
   (ii) ART DES MOLEKÜLS: Peptid
   (ix) MERKMAL:
      (A) NAME/SCHLÜSSEL: Peptide
      (B) LAGE:1..7
      (D) SONSTIGE ANGABEN:/note= "Die Aminoaeuren koennen folgende Bedeutung haben: Pos. 1 (=AS5): Glu, Ser, Asp od. Asn / Bedeutung der anderen Aminosaeuren wie in SEQ.ID Nr. 33 und SEQ.ID Nr. 34"
   (xi) SEQUENZBESCHREIBUNG: SEQ ID NO: 35:
   Xaa Xaa Xaa Xaa Xaa Xaa Xaa
   1               5